# EUROPEAN PATENT SPECIFICATION

(11) **EP 3 471 831 B1**
(45) Date of publication and mention of the grant of the patent: **07.01.2026**
(21) Application number: 16905644.7
(22) Date of filing: 15.06.2016
(51) Int. Cl.: A61Q 3/02, C08F 20/18, C09D 4/02, C09D 133/10

(54) **ONE PART ACRYLIC NAIL FORMULATION**
EINTEILIGE ACRYLISCHE NAGELFORMULIERUNG
FORMULATION D'ONGLE ACRYLIQUE À UNE PARTIE

(43) Date of publication of application: 24.04.2019
(73) Proprietor: Mycone Dental Supply Co., Inc., Gibbstown, NJ 08027 (US); Nail Alliance, LLC, Gladstone, MO 64118 (US)
(72) Inventor: SHERAN, Kevin, Haddonfield New Jersey 08033 (US); HAILE, Danny Lee, La Habra California 90631 (US)
(74) Representative: Boult Wade Tennant LLP
(86) International application number: PCT/US2016/037660
(87) International publication number: WO 2017/217983

(56) References cited:
- WO-A1-2012/024690
- WO-A1-2013/192515
- WO-A1-2015/021237
- WO-A1-2015/046300
- WO-A1-2015/148759
- US-A- 5 102 654
- US-A- 5 407 666
- US-A1- 2006 140 702
- US-A1- 2008 107 695
- US-A1- 2014 242 011
- US-A1- 2015 190 331
- US-A1- 2016 184 213
- US-B1- 6 348 518
- US-B2- 7 981 986
- US-B2- 8 367 045
- US-B2- 8 901 199

## Description

### FIELD OF THE INVENTION

The present invention is directed generally to the field of nail manicuring. More specifically, this invention applies to nail care products and processes for treating nails.

### DESCRIPTION OF RELATED TECHNOLOGY

Acrylic nails are used to artificially enhance the appearance of natural fingernails. Various forms of this product are used throughout the world, including press-on nails, two part (liquid and powder) system, and UV gel system.

Press-on acrylic nails, introduced to the manicure industry in the early 1970s, are nail-shaped pieces of polymer that are glued onto natural nails. Subsequent developments afforded more natural-looking nail enhancements which bond to the real nail by an acrylic-based resin. Such a resin is created by mixing a liquid and powder together to form a thick paste. A nail technician applies the paste over the natural nail and allows it to harden to form a durable nail coating finish that is filed into the desired shape.

Although press-on acrylic nails are used today, they have been largely replaced by sculpted acrylic nails. Sculpted acrylic nails may be formed by several different methods, but the most popular and widely-used method comprises an application of a two-part acrylic formulation by a nail technician. The nail technician typically wets a brush with a monomer liquid, dips the wetted brush into a polymeric powder to form a wet ball of dough, places the wetted ball of the blend of monomer liquid and polymer powder onto a nail and an acrylic nail form adjacent to a nail, and shapes the artificial nail.

The acrylic nail form is a substrate used for construction of acrylic nails beyond the existing free edge of the nail. Elongation requires affixing a substrate means to the exposed edge of the nail, which means has the general contour of the natural nail and extends therefrom to the desired length and along the plane of the natural nail. The substrate is typically of a material to which the polymerized mixture does not adhere, so that the form may be removed following construction of the artificial nail.

The powder portion of the two-part acrylic formulation generally comprises a solid polymer, such as poly (methyl methacrylate), poly (ethyl methacrylate), copolymers of poly (methyl methacrylate) and poly (ethyl methacrylate), and a catalyst, such as benzoyl peroxide.

The two-part acrylic formulation also comprises a liquid, generally referred to as "monomer liquid," which comprises an (meth)acrylic monomer, such methyl methacrylate or ethyl methacrylate, crosslinkers, additives, catalysts such as amine catalysts, and optionally other ingredients needed for forming a mixture with the solid polymer to prepare an artificial nail.

One of the biggest disadvantages of the monomer liquid used in the traditional system is the offensive odor of the ethyl methacrylate. This odor may be unappetizing to most clients and its long term exposure has been hypothesized to be dangerous to the nail technicians.

Odorless systems comprising hydroxyethyl methacrylate, hydroxypropyl methacrylate and other methacrylates have been developed, but they generally have the disadvantage of poor cure and finished aesthetics.

Another method of forming gel nails is by the use of a UV-curable system. In one prior art method of the UV-curable system method, a brush dipped into the formulated monomer liquid is placed into the formulated polymer powder, applied to the client's finger nail, and after the artificial nails are formed, the artificial nails are cured under a UV lamp for several minutes.

A UV-cured system has no odor compared to traditional acrylic systems. However, many nail technicians find that UV-curable systems are difficult to work with and are presently typically only used to create overlays, and not free standing nail extensions. Further, another disadvantage of typical UV-cured two-part powder systems are brittleness of the formed nail.

Gel systems, in contrast to the traditional polish and other polymer-type systems, particularly ultraviolet-cured gel systems, often comprise a gel that may be brushed onto the nails, cured, and shaped to create lifelike artificial nails. As compared with traditional polishes or other non-gel polymer-type systems, gel systems are relatively easy to use, are applicable in less time, are lightweight on the nail, have no odor (or only minimal odor), are durable, and have a high quality shine.

A method of preparation of radiation-curable artificial nail gels is taught in U.S. Patent No. 8,367,045. The method disclosed comprises preparing colored UV-curable artificial nail gel compositions comprising dispersing a pigment in an organic liquid to form a pigment concentrate and mixing the pigment concentrate with a polyfunctional acrylic monomer and/or a polyfunctional acrylic oligomer, resulting in highly colored artificial nail gels.

Composition having a reduced exotherm in actinic curing of urethane (meth)acrylate oligomers on fingernails are disclosed in U.S. Patent No. 9,044,405. A nail coating composition having a reduced exotherm during actinic curing of the coating on a nail may be formed by the use of urethane(meth)acrylate. The composition may include a curable resin, a monomer, a photoinitiator, a chemical filter capable of absorbing ultraviolet (UV) light and reducing exotherm, and an additive.

Compositions for gel applications for nails comprising methacrylates, urethanes, and esters is disclosed in U.S. Patent No. 9,023,326. A radiation curable gel nail coating composition according to that patent comprises about 50 wt% di-[hydroxyethyl methacrylic] trimethylhexyl dicarbamate, about 3 wt% methacrylic acid ester, about 3 wt% hydroxyethyl methacrylate, about 3 wt% hydroxypropyl methacrylate, and about 0.2 wt% of a photoinitiator.

Compositions and methods for UV-curable cosmetic nail coatings is disclosed in U.S. Patent No. 8,901,199. Compositions for natural and artificial nail coatings that provide improved adhesion-promoting and improved solvent-susceptibility may comprise a polymerizable (meth)acrylate, a urethane (meth)acrylate resin and an adhesion promoter selected such as hydroxypropyl methacrylate, hydroxyethyl methacrylate, ethyl methacrylate, tetrahydrofurfuryl methacrylate, and like.

Nail enamel composition containing a urea-modified thixotropic agent in a solvent system are disclosed in U.S. Patent No. 6,555,096. A nail enamel composition which contains, in a cosmetically acceptable solvent system containing diacetone alcohol and at least one additional solvent chosen from C₁-C₆ alkyl acetates and C₁-C₆ alkyl alcohols, at least one film-forming substance and at least one urea-modified thixotropic agent. The use of this thixotropic agent in the specific solvent system gives nail enamel compositions with higher gloss, high clarity, improved aesthetics in the bottle, excellent thixotropic properties, and improved application properties.

WO2013/192515A1 discloses a radiation-curable gel nail coating composition comprising (a) one or more of the following: i. a vinyl functional, urethane; ii. a vinyl containing polyester; and iii. a compound containing an ester which does not contain a hydroxyl group; (b) at least one hydroxyl-containing monomer, oligomer, or solvent; and (c) a photo initiator; the radiation-curable gel nail coating composition comprising less than 10 ppm tin; and a method comprising applying the composition to a finger or toe nail and curing it in the presence of UV radiation.

US63481518B1 discloses an artificial fingernail that is made by forming a liquid component comprising a polymerization promoter and an ester compound; forming a powder component comprising an alkylmethacrylate polymer and a polymerization catalyst; contacting the liquid and powder components; and allowing the composition to polymerize.

US2015/0190331A1 discloses a gel composition useful as a nail lacquer composition, comprises aliphatic urethane acrylate oligomer, polyester acrylate oligomer, low-irritating acrylate monomer, and photoinitiator.

WO2015/046300A1 is directed to composition used for forming artificial nail and nail art kit, comprises polyurethane (meth)acrylate having polycarbonate structure, binder polymer having specified value of weight average molecular weight, and photoinitiator.

Although many advances in the art of formulating acrylic nail system have been made to solve various problems, overcoming problems associated with UV cured systems remain elusive.

### SUMMARY OF THE INVENTION

The present invention relates to a photopolymerizable composition for forming a cosmetic coating for nails comprising: (a) about 50 wt% to about 60 wt% of powder of one or more poly(C₁₋₁₂alkyl(meth)acrylate) polymers; (b) about 25 wt% to about 35 wt% of one or more (meth)acrylate oligomers, wherein the functionality of a (meth)acrylate oligomer is about 2 to about 30; (c) about 10 wt% to about 15 wt% of one or more (meth)acrylate monomers; and (d) one or more crosslinkers; wherein all wt% are with respect to the photopolymerizable composition. This composition exhibits high viscosity that resembles the viscosity of a typical traditional two part system about 4 minutes after the monomer liquid and the polymer have been mixed. This composition can be shaped readily by a nail technician to form nail coatings without any additional use of monomer liquid, solvent, oil, or mixtures thereof, followed by an exposure to UV light to photopolymerize the composition to create a hard, durable nail coating. Optionally, a brush being used for shaping can be wetted with a liquid such as a monomer liquid, solvent, oil, slip agent, or mixtures thereof. Additional ingredients such as colorants and antioxidants may be included in the composition of the present invention.

The composition of the present invention solves one or more problems associated with the formation of a nail coating or nail extension using either procedures relying on gel nail formulations or acrylic nail formulations. Advantages include: a high viscosity composition which is easy to apply either in the neat form or as a mixture with a liquid; the composition of the present invention remains firm and does not run; one part formulation that does not need to be mixed reducing the time serving the client; elimination of any problems about getting the exact mixing ratios correct in order to get a workable composition; the lack of heterogeneity in the formulations which may be associated with two-part formulations; a lack of odor of the one-part formulation; a lower exotherm profile compared to the gel formulations mitigating unpleasantness or pain to the client; the ability able to apply the composition in thicker layers when creating nail extensions; excellent adhesion, abrasion resistance, and mechanical strength of the cured nail coating; self-leveling properties of the one-part formulation over the traditional acrylic nail systems; no significant shrinkage; stability of the one-part formulation; reduction of the yellowing of the cosmetic nail coating; and the very long open time to sculpt the nail.

The composition of the present invention comprises (a) about 50 wt% to about 60 wt% of powder of one or more poly(C₁₋₁₂alkyl(meth)acrylate) polymers. The polymer is a powder and resembles the polymer powder that is typically used in the acrylic nail industry. The composition of the present invention comprises one or more poly(alkyl (meth)acrylates, wherein the alkyl groups comprise one to twelve carbon atoms. The polymer powder consists of fine microspheres with a particle size of 1 to 100 µm, or less than 50 µm, or less than 10 µm, or less than 5 µm.

The photopolymerizable composition of the present invention for forming a cosmetic coating for nails also comprises one or more of about 25 wt% to about 35 wt% of (meth)acrylate oligomers. Examples of (meth)acrylate oligomers include urethane (meth)acrylates, polyester (meth)acrylates, polyester urethane (meth)acrylates, polyether urethane (meth)acrylates, polybutadiene urethane (meth)acrylates, (meth)acrylated polyesters, (meth)acrylated polyethers, (meth)acrylated polyacrylates, and a mixture of any of the foregoing.

The photopolymerizable composition of the present invention for forming a cosmetic coating for nails also comprises about 10 wt% to about 15 wt% of one or more (meth)acrylate monomers. The (meth)acrylate monomer used in the present invention may be any acrylate monomer or methacrylate monomer that is used in nail art formulations in which the curing is performed by UV light. The (meth)acrylate monomer has a formula CH₂=C(R)-COOR', wherein R is H, Me, or a mixture thereof, and R' is an organic group. Under one embodiment of the present invention, the at least one of the one or more (meth)acrylate monomers comprises a hydroxyl-containing (meth)acrylate monomer.

The photopolymerizable composition of the present invention for forming a cosmetic coating for nails also comprises one or more crosslinkers. Such crosslinkers include acrylates, methacrylates, alkoxylated crosslinker, with the formula (CH₂=CMe-C(O)-O-(AO)ₓ-CH₂-)₃C-R; wherein wherein R is a C₁ to C₆ alkyl group; AO is a small alkoxy group; and wherein for each (CH₂=CMe-C(O)-O-(AO)ₓ-CH₂-) group *x* is independently 0, 1, 2, or 3.

In some embodiments, the photopolymerizable composition for forming a cosmetic coating for nails also comprises one or more photoinitiators. The photoinitiator is selected so that it is activated by photons of the wavelength associated with UV light of the UV lamp. Preferably, the photoinitiator should be active at the wavelength of UV light of UV lamps commonly found in nail salons. Such photoinitiators may be selected from benzyl ketones, monomeric hydroxyl ketones, polymeric hydroxyl ketones, α-amino ketones, acyl phosphine oxides, metallocenes, benzophenone, and benzophenone derivatives.

The photopolymerizable composition of some embodiments of the present invention may further comprise a small amount of pigment. Advantages of using a pigment include: providing a tint or a color to the formed cosmetic nail coating; using a pigment is to give a clear or colorless or natural appearance of the cosmetic nail coating; providing a natural appearance to the photopolymerizable composition.

The viscosity of the photopolymerizable composition of some embodiments of the present invention is above 400,000 millipascal-seconds at 25 °C as determined by a cone and plate rheometer using 40 mm 1° steel cone with the truncation gap of 31 microns operating at 2 to 10 s⁻¹ shear rates on a 500 mg sample.

The photopolymerizable composition of some embodiments of the present invention may be prepared by any means used to add and blend high viscosity compositions. Addition of the components, blending of the components and filling appropriate containers may be done at elevated temperatures.

The container in which the photopolymerizable composition of the present invention is sold may be any container capable of handling high viscosity compositions and of providing protection from UV light. Examples of suitable containers include jars or pots, a tube container, and a syringe container. Each of these exemplified containers has an orifice which is designed to deliver a bead of the photopolymerizable composition. The orifice is designed so that the technician is able to deliver a uniform bead to the nail of the client.

Another aspect of the present invention is the method of use of the above-described photopolymerizable composition to form a cosmetic coating for a nail. There are three methods which may be used to form the cosmetic nail coating. The first method of forming the cosmetic nail coating comprises the steps of placing the above-described composition onto a nail of the client; and exposing the composition to UV light. The second method is similar to the first method except that it is preceded with a wetting of the composition. The third method is also similar to the first method, except that the wetting is done after the composition is placed on the nail.

After the composition is shaped, the composition, along with finger and the hand of the client, is exposed to UV light to cure the composition. The liquid of this method is selected from the group consisting of an acrylic nail monomer, solvent, oil, slip agent, and mixtures thereof.

The invention is defined by the appended claims. Also described herein are further examples useful for understanding the invention.

In the first example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition.

In the second example described herein isa photopolymerizable composition for forming a cosmetic coating for nails comprising: (a) about 50 wt% to about 60 wt% of one or more poly(C₁₋₁₂alkyl(meth)acrylate) polymers; (b) about 25 wt% to about 35 wt% of one or more (meth)acrylate oligomers; (c) about 10 wt% to about 15 wt% of one or more (meth)acrylate monomers; and (d) one or more crosslinkers; wherein all wt% are with respect to the photopolymerizable composition.

In the third example described herein is a photopolymerizable composition for forming a cosmetic coating for nails comprising: (a) about 50 wt% to about 60 wt% of one or more poly(C₁₋₁₂alkyl(meth)acrylate) polymers; (b) about 25 wt% to about 35 wt% of one or more (meth)acrylate oligomers comprising a urethane (meth)acrylate oligomer; (c) about 10 wt% to about 15 wt% of one or more (meth)acrylate monomers comprising a monomer selected from the group consisting of hydroxyalkyl (meth)acrylate, cycloalkyl (meth)acrylate, and a mixture thereof; and (d) one or more crosslinkers; wherein all wt% are with respect to the photopolymerizable composition.

In the fourth example described herein is a photopolymerizable composition for forming a cosmetic coating for nails comprising: (a) about 50 wt% to about 60 wt% of one or more poly(C₁₋₁₂alkyl(meth)acrylate) polymers; (b) about 25 wt% to about 35 wt% of one or more (meth)acrylate oligomers comprising a urethane (meth)acrylate oligomer; (c) about 10 wt% to about 15 wt% of one or more (meth)acrylate monomers comprising a monomer selected from the group consisting of hydroxyalkyl (meth)acrylate, cycloalkyl (meth)acrylate, and a mixture thereof; (d) one or more crosslinkers comprising a tri((meth)acrylate); and further comprising a photoinitiator; wherein all wt% are with respect to the photopolymerizable composition.

In the fifth example described herein is a photopolymerizable composition for forming a cosmetic coating for nails comprising: (a) about 20 wt% to about 80 wt% of one or more polymers selected from the group consisting of poly(methyl methacrylate), poly(ethyl methacrylate), styrene/acrylate copolymer, styrene/acrylate/divinylbenzene copolymer, and a mixture thereof; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition.

In the sixth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof, comprising poly(methyl methacrylate) particles, wherein the mean particle size of poly(methyl methacrylate) particles is less than about 100 micrometers; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition.

In the seventh example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof, comprising poly(methyl methacrylate) particles, wherein the mean particle size of poly(methyl methacrylate) particles is less than about 50 micrometers; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition.

In the eighth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof, comprising poly(methyl methacrylate) particles, wherein the mean particle size of poly(methyl methacrylate) particles is less than about 10 micrometers; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition.

In the ninth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof, comprising poly(methyl methacrylate) particles, wherein the mean particle size of poly(methyl methacrylate) particles is less than about 5 micrometers; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition.

In the tenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers wherein at least one of the one or more the (meth)acrylate oligomer is selected from the group consisting of urethane (meth)acrylate, epoxy (meth)acrylate, epoxy urethane (meth)acrylate, (meth)acrylated acrylate, (meth)acrylated polyether, (meth)acrylated polycarbonate, (meth)acrylated cellulose, (meth)acrylated butadiene, (meth)acrylated styrene, polyester (meth)acrylate, polyester urethane (meth)acrylate, polyether urethane (meth)acrylate, polybutadiene urethane (meth)acrylate, and a mixture thereof; (c) about 5 wt% to about 60 wt% of one or more (meth)acrylate monomers; and (d) one or more crosslinkers; wherein all wt% are with respect to the photopolymerizable composition.

In the eleventh example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers wherein at least one of the one or more (meth)acrylate monomers comprises a hydroxyl-containing (meth)acrylate monomer; and (d) one or more crosslinkers; wherein all wt% are with respect to the photopolymerizable composition.

In the twelfth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers wherein at least one of the one or more (meth)acrylate monomers comprise a methacrylate selected from the group consisting of hydroxypropyl methacrylate, hydroxyethyl methacrylate, ethyl methacrylate, propyl methacrylate, butyl methacrylate, isobutyl methacrylate, sec-butyl methacrylate, tetrahydrofurfuryl methacrylate, caprolactone methacrylate, methacroyloxyethyl maleate, 2-hydroxyethyl methacrylate/succinate, phthalic acid monoethyl methacrylate, or a mixture thereof; and (d) one or more crosslinkers; wherein all wt% are with respect to the photopolymerizable composition.

In the thirteenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers wherein at least one of the one or more crosslinkers is selected from the group consisting of tri(meth)acrylate, tetra(meth)acrylate, penta(meth)acrylate, and a mixture thereof; wherein all wt% are with respect to the photopolymerizable composition.

In the fourteenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers, wherein at least one of the one or more crosslinker is an alkoxylated crosslinker of formula (CH₂=CMe-C(O)-O-(AO)ₓ-CH₂-)₃C-R; wherein R is a C₁ to C₆ alkyl group; AO is an alkoxy group selected from the group consisting of ethylene oxide, -CH₂-CH₂-O-, propylene oxide, -CH(CH₃)-CH₂-O-, -CH₂-CH₂-CH₂-O-, butylene oxide, and -CH(Et)-CH₂-O-; and wherein for each CH₂=CMe-C(O)-O-(AO)ₓ-CH₂- group *x* is independently 0, 1, 2, or 3; wherein all wt% are with respect to the photopolymerizable composition.

In the fifteenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers, wherein at least one of the one or more crosslinker is ethoxylated crosslinker of formula (CH₂=CMe-C(O)-O-(CH₂-CH₂-O)ₓ₋CH₂-)₃C-C₂H₅; wherein for each CH₂=CMe-C(O)-O-(CH₂-CH₂-O)ₓ-CH₂- group *x* is independently 0, 1, 2, or 3; wherein all wt% are with respect to the photopolymerizable composition.

In the sixteenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers, wherein at least one of the one or more crosslinker is selected from the group consisting of trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, dipentaerythritol penta(meth)acrylate and ethoxylated iscyanuric acid tri(meth)acrylates; wherein all wt% are with respect to the photopolymerizable composition.

In the seventeenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; (d) one or more crosslinkers; and (e) one or more photoinitiators; wherein all wt% are with respect to the photopolymerizable composition.

In the eighteenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; (d) one or more crosslinkers; and (e) one or more photoinitiators, wherein at least one of the one or more photoinitiators is selected from the group consisting of 1-hydroxy-cyclohexylphenylketone; benzophenone; 2-benzyl-2-(dimethylamino)-1-(4-(4-morphorlinyl)phenyl)-1-butanone; 2,2-dimethoxy-2-phenyl acetophenone; 2-methyl-1-(4-methylthio)phenyl-2-(4-morphorlinyl)-1-propanone; 2,4,6-trimethylbenzoyldiphenylphosphine oxide; bis(2,4,6-trimethyl benzoyl)phenyl phosphine oxide; diphenyl-(2,4,6-trimethylbenzoyl) phosphine oxide; bis(2,6-dimethoxybenzoyl-2,4,4-trimethyl pentyl)phosphine oxide; 2-hydroxy-2-methyl-1-phenyl-1-propanone; phenyl bis(2,4,6-trimethylbenzoyl) phosphine oxide; benzyl-dimethylketal; isopropylthioxanthone; bis(η5-2,4-cyclopentadien-1-yl)bis[2,6-difluoro-3-(1H-pyrrol-1-yl)phenyl]titanium); α,α-dimethoxy α-phenyl acetophenone; ethyl (2,4,6-trimethyl benzoyl) phenyl phosphinate; phenyl (2,4,6-trimethyl benzoyl) phenyl phosphinate, methyl benzoyl formate, and mixtures thereof; wherein all wt% are with respect to the photopolymerizable composition.

In the nineteenth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers; further comprising a colorant or special effects pigment or a combination thereof, wherein all wt% are with respect to the photopolymerizable composition.

In the twentieth example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers; further comprises a colorant or special effects pigment or a combination thereof, wherein the colorant is selected from the group consisting of ultramarine, manganese violet, zinc oxide, FD&C Blue No. 1, D&C Blue No. 4, Iron Blue, D&C Violet No. 2, and a mixture thereof; wherein all wt% are with respect to the photopolymerizable composition.

In the twenty-first example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition, wherein the viscosity of the photopolymerizable composition is greater than 400,000 millipascal-seconds at 25 °C as determined by a cone and plate rheometer using 40 mm 1° steel cone with the truncation gap of 31 microns operating at 10 s⁻¹ shear rates on a 500 mg sample.

In the twenty-second example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition, wherein a 0.5 gram sample of the photopolymerizable composition exhibits a temperature increase of less than about 20 °C during the exposure to UV light.

In the twenty-third example described herein is a photopolymerizable composition for forming a cosmetic coating for nails comprising: (a) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (b) about 15 wt% to about 80 wt% of one or more (meth)acrylate oligomers; (c) about 5 wt% to about 60 wt% of one or more (meth)acrylate monomers; and (d) one or more crosslinkers; wherein all wt% are with respect to the photopolymerizable composition, wherein the composition is stable in a dark container at 49 °C for four months, or at 65 °C for 2 weeks.

In the twenty-fourth example described herein is a method of forming a cosmetic nail coating comprising the steps of: (a) placing a photopolymerizable composition for forming a cosmetic coating for nails comprising: (i) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (ii) about 15 wt% to about 80 wt% of one or more (meth)acrylate oligomers; (iii) about 5 wt% to about 60 wt% of one or more (meth)acrylate monomers; (iv) one or more crosslinkers; and (v) one or more photoinitiators; wherein all wt% are with respect to the photopolymerizable composition onto a nail; and (b) exposing the composition to UV light.

In the twenty-fifth example described herein is a method of forming a cosmetic nail coating comprising the steps of: (a) contacting a photopolymerizable composition for forming a cosmetic coating for nails comprising: (i) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (ii) about 15 wt% to about 80 wt% of one or more (meth)acrylate oligomers; (iii) about 5 wt% to about 60 wt% of one or more (meth)acrylate monomers; and (iv) one or more crosslinkers; wherein all wt% are with respect to the photopolymerizable composition with a liquid selected from the group consisting of an acrylic nail monomer, solvent, oil, slip agent, photoinitiator and mixtures thereof to create a blend; (b) placing the blend onto a nail; and (c) exposing the blend to UV light.

In the twenty-sixth example described herein is a method of forming a cosmetic nail coating comprising the steps of: (a) placing a photopolymerizable composition for forming a cosmetic coating for nails comprising: (i) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (ii) about 15 wt% to about 80 wt% of one or more (meth)acrylate oligomers; (iii) about 5 wt% to about 60 wt% of one or more (meth)acrylate monomers; and (iv) one or more crosslinkers; wherein all wt% are with respect to the photopolymerizable composition onto a nail; (b) contacting the composition with a liquid selected from the group consisting of an acrylic nail monomer, solvent, oil, photoinitiator and mixtures thereof; and (c) exposing the mixture to UV light.

In the twenty-seventh example described herein is a container containing the composition of a photopolymerizable composition for forming a cosmetic coating for nails comprising: (i) about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof; (ii) about 15 wt% to about 80 wt% of one or more (meth)acrylate oligomers; (iii) about 5 wt% to about 60 wt% of one or more (meth)acrylate monomers; and (iv) one or more crosslinkers; wherein all wt% are with respect to the photopolymerizable composition, wherein the container is a tube container or a syringe container, and wherein the container delivers a uniform bead of the composition.

### DETAILED DESCRIPTION OF THE INVENTION

For illustrative purposes, the principles of the present invention are described by referencing various exemplary embodiments thereof. Although certain embodiments of the invention are specifically described herein, one of ordinary skill in the art will readily recognize that the same principles are equally applicable to, and can be employed in other apparatuses and methods. Before explaining the disclosed embodiments of the present invention in detail, it is to be understood that the invention is not limited in its application to the details of any particular embodiment shown. The terminology used herein is for the purpose of description and not of limitation. Further, although certain methods are described with reference to certain steps that are presented herein in certain order, in many instances, these steps may be performed in any order as may be appreciated by one skilled in the art, and the methods are not limited to the particular arrangement of steps disclosed herein.

As used herein and in the appended claims, the singular forms "a", "an", and "the" include plural references unless the context clearly dictates otherwise. The singular form of any class of the ingredients refer not only to one chemical species within that class, but also to a mixture of those chemical species; for example, the term " photoinitiator" in the singular form, may refer to a mixture of compounds each of which is also a photoinitiator. The terms "a" (or "an"), "one or more" and "at least one" can be used interchangeably herein. It is also to be noted that the terms "comprising", "including", and "having" can be used interchangeably.

The abbreviations and symbols as used herein, unless indicated otherwise, take their ordinary meaning. The abbreviation or symbol "cp" means centipoises, or millipascal-seconds. The abbreviation or symbol "µm" means micrometer. The term "about" when referring to a number means ±3%. For example, the phrase "about 50 wt%" refers to a number between and including 48.500 wt% and 51.500 wt%.

The term "wt%" means percent by weight. The phrase "wherein all wt% are with respect to the photopolymerizable composition" means that the recited percent by weight values for each ingredient is compared to the entire photopolymerizable composition. The entire photopolymerizable composition includes not only ingredients which wt% are listed, but also listed ingredients for which wt% are omitted, and also ingredients that may not be recited.

The term "client" refers to a person whose nails are being treated.

The phrase "nail technician" or "technician" is a worker skilled or licensed in the art of providing nail extensions, artificial nails, acrylic nails, gel nails, and other manicure services for clients. Alternative names for a nail technician may include a manicurist, or a cosmetologist. Such a person may work for pay at a nail salon, or may be a manicure aficionado.

Under one embodiment of the present invention, the client and the nail technician are two different individuals. Although the description of the invention below describes the nail technician and the client as two separate individuals, it is understood that the claimed invention and methods are also suitable for use by a single person who is both a nail technician and a client. Under another embodiment of the present invention, the client and the nail technician are the same person.

The terms "nail", refer to either a fingernail or a toenail. The term "nail" also refers to a human nail, as well as to any toughened keratin at the end of a digit of a non-human animal. The phrase "cosmetic nail coating" refers to the hardened, fully cured substance covering a part or all of the nail, and any portions of this substance that extends or is built beyond the free edge of the nail.

The term "acrylic" in the phrase "acrylic nail" refers to hardened polymerized composition used in manicure arts, which are composed of any of several types of poly ((meth)acrylates), or copolymers of various (meth)acrylate monomers, oligomers or copolymers of various (meth)acrylate monomers with any of several non-(meth)acrylitic monomers.

When referring to a composition, the definition of the term "acrylate" as referred to in the monomeric form, includes an ester, a salt, or a conjugate base of acrylic acid, with the formula CH₂=CH-COO⁻. The definition of the term "acrylate" referred to in the polymeric or oligomeric form includes the repeating unit of an ester, a salt, or a conjugate base of acrylic acid, with the formula -[CH₂-CH(COO⁻)]-.

The definition of the term "methacrylate" as referred to in the monomeric form includes an ester, a salt, or a conjugate base of methacrylic acid, with the formula CH₂=C(CH₃)-COO⁻. The definition of the term "methacrylate" as referred to in the polymeric or oligomeric form includes an ester, a salt, or a conjugate base of methacrylic acid, with the formula -[CH₂=C(CH₃)-COO⁻]-.

The term "(meth)acrylate" means acrylate, methacrylate, or a mixture thereof. When referring to a compound, "(meth)acrylate" means an ester, a salt, or a conjugate base of an acrylic acid, with the formula CH₂=C(R)-COO⁻, wherein R is H, Me, or a mixture thereof. The definition of the term "(meth)acrylate" as referred to in the polymeric or oligomeric form includes an ester, a salt, or a conjugate base of methacrylic acid, with the formula -[CH₂=C(R)-COO⁻]-, wherein R is H, Me, or a mixture thereof. By extension, a monomer, oligomer, or polymer name containing as a part of its term the string "(meth)acrylate" should be interpreted as referring to the same monomer, oligomer, or polymer, that is an acrylate, methacrylate, or a mixture thereof. For example, the term "poly(C₁₋₁₂alkyl (meth)acrylate)" means "any of poly(C₁₋₁₂alkyl acrylate), poly(C₁₋₁₂alkyl methacrylate), and a mixture of poly(C₁₋₁₂alkyl acrylate) and poly(C₁₋₁₂alkyl methacrylate)".

The present invention relates to a photopolymerizable composition for forming a cosmetic coating for nails comprising: (a) about 50 wt% to about 60 wt% of powder of one or more poly(C₁₋₁₂alkyl(meth)acrylates); (b) about 25 wt% to about 35 wt% of one or more (meth)acrylate oligomers, wherein the functionality of a (meth)acrylate oligomer is about 2 to about 30; (c) about 10 wt% to about 15 wt% of one or more (meth)acrylate monomers; (d) one or more crosslinkers; and (e) optionally, one or more photoinitiators; wherein all wt% are with respect to the photopolymerizable composition.

This composition exhibits high viscosity that resembles the viscosity of a typical traditional two part system about 4 minutes after the monomer liquid and the polymer have been mixed. This composition can be shaped readily by a nail technician to form nail coatings without any additional use of monomers liquid, followed by an exposure to UV light to photopolymerize the composition to create a hard, durable nail coating.

The composition of the present invention comprises at least a polymer, an oligomer, a monomer, a crosslinker, and optionally a photoinitiator. Each or any of these five ingredients may be a single compound, or each or any of these ingredients may be a mixture of compounds that fall within the definition of such ingredient. Additional ingredients such as a colorant, a special effects pigment or an antioxidant may be included in the composition of the present invention.

The composition of the present invention solves one or more problems associated the formation of nail coating or nail extension using either procedures relying on gel nail formulations or acrylic nail formulations.

One of the advantages that the present invention provides is a high viscosity composition which is easy to apply. Compared to the low viscosity of nail gel formulations that may run outside of the sculpting area, the composition of the present invention has a high viscosity out of the container. The composition of the present invention does not move easily until it is pushed into a desired shape by a manicurist tool, such as a brush or a pusher. The pushing of the composition of the present invention into a desired shape may be done neat, or it may be done with the aid of liquid which lowers the viscosity of the composition. One advantage is that either in the neat form or as a mixture with a liquid, the composition of the present invention remains firm and does not run. The technician may optionally control the viscosity through the use of the suitable liquid applied until the composition of the present invention is cured by ultraviolet light. Thus, total control over the viscosity of the nail covering or nail extension may be attained.

Another advantage of the composition of the present invention and the method of its using over the traditional acrylic nail formulation is that the composition is a one part formulation. There is no need to mix the composition of the present invention with another part. By not spending time mixing the formulation or waiting for it to set to a workable viscosity, time serving the client is reduced, resulting in faster service. Further, the technician does not have to worry about getting the exact mixing ratios correct in order to get a workable composition.

Another advantage of the one-part formulation is the lack of heterogeneity in the formulations which may be associated with two-part formulations. The composition of the present invention is homogeneous, allowing for uniform applicability of the composition.

A further advantage of the composition of the present invention is a lack of odor of the one-part formulation. Traditional two-part acrylic systems typically exhibit very strong odors; for example, a common commercially available monomer composition that comprises a mixture of ethyl methacrylate, glycol HEMA-methacrylate, HEMA, benzophenone-1, and dimethyltolylamine, exhibits a severely strong odor. The composition of the present invention has little or no odor. This is especially desirable in small nail salons and spas, where multiple nail technicians may work side by side for many hours in a tight environment, or in environments with limited air circulation.

Yet a further advantage of the present invention is the lower exotherm profile compared to the gel formulations. UV light initiated gel formulas create heat when curing. Such heat can be unpleasant or even painful to the client. This is especially true for if the product is applied in a thick layer, which results in an increase of the heat flux from the gel into the nail bed. It has been observed that the composition of the present invention exhibits a significantly lower exotherm of the polymerization reaction. Because of the lack of such a heat spike, the technician is able to apply the composition to the present invention in thicker layers when creating nail extensions.

A still further advantage of the present invention is the excellent adhesion, abrasion resistance, and mechanical strength of the cured nail coating.

Another advantage of the present invention is that unlike gel nail coverings which tend to shrink while curing, the composition of the present invention displays no significant shrinkage.

A further advantage of the composition of the present invention is the stability of the one-part formulation. Because the composition is thermally stable and needs UV light to cure it, the composition of the present invention under one embodiment is shelf stable for several days, under another embodiment is shelf stable for several months, and under still another embodiment is shelf stable for several years.

Yet another advantage of one embodiment of the present invention is the reduction of the yellowing of the cosmetic nail coating prepared from the composition of the present invention. Unlike many other compositions that are thermally cured, or UV cured, the composition of the present invention, when cured, does not appreciably yellow.

Yet still another advantage of the present invention compared to the traditional two-part system is the long open time to sculpt the nail. The composition may be worked by the technician for as long as is necessary before the composition is exposed to UV light.

The above advantages listed above are illustrative and may not be exhaustive. Further, not every embodiment of the present invention necessarily displays all of the advantages listed.

In an example useful for understanding the invention, described herein is a composition that comprises about 20 wt% to about 80 wt% of one or more polymers comprised of the group consisting of poly(C₁₋₁₂alkyl(meth)acrylate) polymer, poly(C₁₋₁₂alkyl(meth)acrylate) copolymer, styrene polymer, styrene copolymer, and mixtures thereof. In another example useful for understanding the invention, described herein is a composition that comprises about 20 wt% to about 80 wt% of polymer selected from the group consisting of poly(C₁₋₁₂alkylacrylate) polymer, poly(C₁₋₁₂alkyl methacrylate) polymer, poly(C₁₋₁₂alkylacrylate) copolymer, poly(C₁₋₁₂alkyl methacrylate) copolymer, styrene polymer, styrene copolymer, and mixtures thereof.

In the composition of the present invention, the composition comprises about 50 wt% to about 60 wt% of one or more poly(C₁₋₁₂alkyl(meth)acrylate) polymer.

The polymer is a powder and resembles the polymer powder that is typically used in the acrylic nail industry. The composition of the present invention comprises a polyalkyl(meth)acrylate, or a mixture thereof, wherein the alkyl groups comprise one to twelve carbon atoms.

When mixed with the other ingredients of the photopolymerizable composition of the present invention, the polymer typically does not dissolve, but forms a homogenous suspension of the polymer in the composition.

The phrase "poly(C₁₋₁₂alkyl(meth)acrylate) polymer" means a polymer which is comprised mostly or exclusively of C₁₋₁₂alkyl(meth)acrylate monomer residues, or polymer units. Under one embodiment most or all of the C₁₋₁₂alkyl(meth)acrylate polymer units are the same. Under another embodiment, the C₁₋₁₂alkyl(meth)acrylate polymer units are different from each other. An example of "poly(C₁₋₁₂alkyl(meth)acrylate) polymer" includes a polymer that comprises methyl acrylate units, methacrylate units, and methyl methacrylate units.

The phrase "poly(C₁₋₁₂alkyl(meth)acrylate) copolymer" means a copolymer of C₁₋₁₂alkyl(meth)acrylate polymer units with other polymer units. The C₁₋₁₂alkyl(meth)acrylate polymer units may be the same or they may vary. The phrase "other polymer units" means polymer units that do not fall within the definition of poly(C₁₋₁₂alkyl(meth)acrylate); examples of such, include styrene, divinyl benzene, and a mixture of styrene and divinyl benzene.

Under one example, the C₁₋₁₂alkyl(meth)acrylate polymer units in the poly(C₁₋₁₂alkyl(meth)acrylate) copolymer are the same units. Under another example, the C₁₋₁₂alkyl(meth)acrylate polymer units in the poly(C₁₋₁₂alkyl(meth)acrylate) copolymer are different polymer units. An example of "poly(C₁₋₁₂alkyl(meth)acrylate) copolymer" includes a polymer that comprises methyl acrylate units, methyl methacrylate units, and styrene units.

The phrase "styrene polymer" means a polymer consisting essentially of -[CH₂-CHPh]- polymer units. The phrase "polymer unit" is synonymous with a "monomer residue", or with a "repeating unit".

The phrase "styrene copolymer" means a copolymer of styrene units with other polymer units that are not styrene. Examples of "styrene copolymer" includes styrene/(meth)acrylate copolymer, and styrene/(meth)acrylate/divinylbenzene copolymer.

Copolymer comprising divinyl benzene is generally used in amounts as needed for crosslinking. Under one embodiment, the solid polymer comprises little or no crosslinking. Under another embodiment the solid polymer comprises significant amount of crosslinking, which may mitigate the solubility of the polymer.

Examples of poly(C₁₋₁₂alkyl (meth)acrylate) include poly(methyl (meth)acrylate), poly(ethyl (meth)acrylate), poly(propyl (meth)acrylate), poly(n-propyl (meth)acrylate), poly(isopropyl (meth)acrylate), poly(butyl (meth)acrylate), poly(n-butyl (meth)acrylate), poly(isobutyl (meth)acrylate), poly(sec-butyl (meth)acrylate), poly(pentyl (meth)acrylate), poly(hexyl (meth)acrylate), poly(heptyl (meth)acrylate), poly(octyl (meth)acrylate), poly(nonyl (meth)acrylate), poly(decyl (meth)acrylate), poly(undecyl (meth)acrylate), and poly(dodecyl (meth)acrylate). In these examples, the alkyl groups hexyl, heptyl, octyl, nonyl, decyl, undecyl, and dodecyl may be straight chain groups or branched groups.

Under one example the poly(C₁₋₁₂alkyl (meth)acrylate) polymer or copolymer thereof is a copolymer of poly(C₁₋₁₂alkyl (meth)acrylate). Examples of such copolymers include styrene / C₁₋₁₂alkyl (meth)acrylic copolymer, styrene / C₁₋₁₂alkyl (meth)acrylate, styrene / C₁₋₁₂alkyl (meth)acrylate / divinylbenzene copolymers, and styrene/PEG-10 maleate/nonoxynol-10 maleate/acrylate copolymer.

Examples of poly(C₁₋₄alkyl (meth)acrylate) include poly(methyl (meth)acrylate), poly(ethyl (meth)acrylate), poly(propyl (meth)acrylate), poly(n-propyl (meth)acrylate), poly(isopropyl (meth)acrylate), poly(butyl (meth)acrylate), poly(n-butyl (meth)acrylate), poly(isobutyl (meth)acrylate), and poly(sec-butyl (meth)acrylate).

Under one embodiment of the present invention, the polymer consists of a single type of polymer. Under an alternative embodiment, the polymer consists of a mixture of poly(C₁₋₁₂alkyl acrylate) polymers; a mixture of poly(C₁₋₁₂alkyl methacrylate) polymers; a mixture of poly(C₁₋₁₂alkyl acrylate) polymer and poly(C₁₋₁₂alkyl methacrylate) polymer; a mixture of poly(C₁₋₁₂alkyl acrylate) polymers and poly(C₁₋₁₂alkyl methacrylate) polymer; a mixture of poly(C₁₋₁₂alkyl acrylate) polymers and poly(C₁₋₁₂alkyl methacrylate) polymer; a mixture of poly(C₁₋₁₂alkyl acrylate) polymers and poly(C₁₋₁₂alkyl methacrylate) polymers.

Examples of a suitable polymer comprises poly(methyl methacrylate), poly(ethyl methacrylate), and a mixture thereof. A suitable polymer is poly(methyl methacrylate), or PMMA.

Under one embodiment, PMMA is blended with other polymers to improve its flexibility.

The polymer is a powder which may be prepared by a routine technique, such as suspension polymerization in which the reaction takes place between droplets of the corresponding monomer suspended in a solution of water and catalyst.

The molecular weight of the polymer suitable for use in the present invention is similar to the polymers used in the acrylic nail industry.

The polymer powder prior to mixing with other ingredients consists of fine microspheres. Under one embodiment, when mixed with the oligomers, monomers, and other ingredients, these microspheres dissolve completely or partially.

Under another embodiment, when mixed with the oligomers, monomers, and other ingredients, these microspheres are insoluble in the composition. Microspheres are said to be insoluble when less than 5% of the diameter of the microsphere is lost to the solution. Generally, insoluble microspheres gain weight and size as they swell upon exposure to the monomers and other ingredients.

The polymer powder under one embodiment consists of fine microspheres. Exemplary size of the microspheres (*i.e.,* particle size of the powder) is 1 to 100 µm. Under one embodiment of the present invention, the mean particle size is less than 100 µm. Under another embodiment of the present invention, the mean particle size is less than 50 µm. Under still another embodiment of the present invention, the mean particle size is less than 10 µm. Under yet another embodiment of the present invention, the mean particle size is less than 5 µm.

The polymer powder may also further comprise other components such as radical intiators.

The photopolymerizable composition of the present invention for forming a cosmetic coating for nails also comprises about 25 wt% to about 35 wt% of (meth)acrylate oligomer.

Under one embodiment of the present invention, the (meth)acrylate oligomer consists of a single type of oligomer. Under an alternative embodiment, the (meth)acryate oligomers consists of a mixture of oligomers.

The molecular weight of the (meth)acrylate oligomer suitable for use in the present invention is similar to the (meth)acrylate oligomers used in the acrylic nail industry.

The functionality of a (meth)acrylate oligomer is about 2 to about 30.

Examples of (meth)acrylate oligomers include urethane (meth)acrylate, epoxy (meth)acrylate, epoxy urethane (meth)acrylate, (meth)acrylated acrylate, (meth)acrylated polyether, (meth)acrylated polycarbonate, (meth)acrylated cellulose, (meth)acrylated butadiene, (meth)acrylated styrene, polyester (meth)acrylate, polyester urethane (meth)acrylate, polyether urethane (meth)acrylate, polybutadiene urethane (meth)acrylate, and a mixture thereof.

Urethane (meth)acrylate comprises at least two acryl or methacryl groups and at least one urethane group. Urethane (meth)acrylate may also comprise additional functional groups, such as an ester, an ether, and like. The phrase "urethane (meth)acrylate" thus also includes polyester urethane acrylate, polyester urethane methacrylate, polyether urethane acrylate, polyether urethane methacrylate, and like.

Examples of urethane (meth)acrylate oligomers include: aliphatic urethane (meth)acrylates, aromatic urethane (meth)acrylates, polyester urethane (meth)acrylates, and polyether polyols and aliphatic, aromatic, polyester, polyether diisocyanates capped with (meth)acrylate end-groups, epoxy (meth)acrylates, epoxy urethane (meth)acrylates.

Epoxy acrylates and epoxy methacrylates may be based on aliphatic or aromatic epoxy prepolymers capped with acrylate or methacrylate end-groups.

Acrylated polyester oligomers, useful in one embodiment of the present invention, have at least two or more acrylate or methacrylate groups and a polyester core. Acrylated polyether oligomers have at least two or more acryl or methacryl groups and a polyether core. Acrylated acrylate oligomers have at least two or more acryl or methacryl groups and a polyacrylic core.

Urethane (meth)acrylate can be can be prepared by any conventional means, such as one of two modes of addition. A polyol can be terminated on each end with diisocyanate and then capped with a hydroxy functional (meth)acrylate. Alternatively, a diisocyanate can first be reacted with a hydroxy functional (meth)acrylate, and that product can be used to terminate a polyol.

Under one embodiment the oligomers is an aliphatic urethane acrylate. Under an alternative embodiment the oligomers is a polyester urethane acrylate. Under still another embodiment the oligomers is a mixture of is a mixture of an aliphatic urethane acrylate oligomer and a polyester urethane acrylate oligomer.

The photopolymerizable composition of the present invention for forming a cosmetic coating for nails also comprises about 10 wt% to about 15 wt% of one or more (meth)acrylate monomers.

The (meth)acrylate monomer used in the present invention may be any acrylate monomer or methacrylate monomer that is used in nail art formulations in which the curing is performed by UV light. The (meth)acrylate monomer has a formula CH₂=C(R)-COOR', wherein R is H, Me, or a mixture thereof, and R' is an organic group. Examples of organic group R' include hydrocarbons, that is, aliphatic (e.g., alkyl or alkenyl), alicyclic (e.g., cycloalkyl, cycloalkenyl) compounds, and aromatic-, aliphatic-, and alicyclic-substituted aromatic compounds, as well as cyclic compounds wherein the ring is completed through another portion of the molecule (e.g., two substituents together form an alicyclic compound); groups that include hetero atoms, that is, groups that contain other than carbon in a ring or chain otherwise composed of carbon atoms, such as oxygen, nitrogen, such as groups containing non-hydrocarbon groups, such as alkoxy, amino, amido, and similar groups.

Examples of (meth)acrylate include methyl acrylate, methyl methacrylate, ethyl acrylate, ethyl methacrylate, hydroxypropyl acrylate, hydroxypropyl methacrylate, butylacrylate, butyl methacrylate, hydroxyethyl acrylate, propyl methacrylate, isobutyl methacrylate, sec-butyl methacrylate, hydroxyethyl methacrylate, butoxyethyl acrylate, butoxyethyl methacrylate, diethylaminoethyl acrylate, diethylaminoethyl methacrylate, 2-ethylhexyl acrylate, 2-ethylhexyl methacrylate, ethoxyethyl acrylate, ethoxyethyl methacrylate, t-butyl aminoethyl acrylate, t-butyl aminoethyl methacrylate, methoxyethylene glycolacrylate, methoxyethylene glycol methacrylate, phosphoethyl acrylate, phosphoethyl methacrylate, methoxy propyl acrylate, methoxy propyl methacrylate, phenoxyethylene glycol acrylate, tetrahydrofurfuryl methacrylate, phenoxyethylene glycol methacrylate, caprolactone methacrylate, methacroyloxyethyl maleate, 2-hydroxyethyl methacrylate/succinate, phthalic acid monoethyl methacrylate, phenoxypolyethylene glycol acrylate, phenoxypolyethylene glycol methacrylate, 2-hydroxy-3-phenoxypropyl acrylate, 2-hydroxy-3-phenoxypropyl methacrylate, isobornyl acrylate, isobornyl methacrylate, 3-chloro-2-hydroxypropyl acrylate, 3-chloro-2-hydroxypropyl methacrylate, and mixtures thereof.

Under one embodiment, the at least one or more (meth)acrylate monomers comprise a methacrylate selected from the group consisting of hydroxypropyl methacrylate, hydroxyethyl methacrylate, ethyl methacrylate, propyl methacrylate, butyl methacrylate, isobutyl methacrylate, sec-butyl methacrylate, tetrahydrofurfuryl methacrylate, caprolactone methacrylate, methacroyloxyethyl maleate, 2-hydroxyethyl methacrylate/succinate, phthalic acid monoethyl methacrylate, or a mixture thereof.

Under one embodiment of the present invention, the at least one of the one or more (meth)acrylate monomers comprises a hydroxyl-containing (meth)acrylate monomer.

The photopolymerizable composition of the present invention for forming a cosmetic coating for nails also comprises one or more crosslinkers. A crosslinker is a compound that contains two or more (meth)acrylate groups. Crosslinkers are necessary to provide chemical bonding between several monomers, oligomers, polymers or combinations thereof to yield a cross-linked polymeric structure needed for the formation of cosmetic nail coating.

Under one embodiment the monomer liquid comprises a single crosslinker. Under another embodiment the monomer liquid comprises two or more different compounds that are crosslinkers.

Examples of crosslinkers include diacrylates, triacrylates, tetraacrylates, pentaacrylates and higher acrylates. Such examples include trimethylolpropane triacrylate, trimethylolethane triacrylate, trimethylolpropane trimethacrylate, trimethylolethane trimethacrylate, tetramethylene glycol dimethacrylate, triethylene glycol dimethacrylate, tetraethylene glycol diacrylate, pentaerythritol diacrylate, penta-erythritol triacrylate, pentaerythritol tetraacrylate, dipentaerythritol diacrylate, dipenta-erythritol triacrylate, dipentaerythritol tetraacrylate, dipentaerythritol pentaacrylate, dipenta-erythritol hexaacrylate, tripentaerythritol octaacrylate, pentaerythritol dimethacrylate, penta-erythritol trimethacrylate, dipentaerythritol dimethacrylate, dipentaerythritol tetramethacrylate, tripentaerythritol octamethacrylate, ethylene glycol diacrylate, 1,3-butanediol diacrylate, 1,3-butanediol dimethacrylate, sorbitol triacrylate, sorbitol tetraacrylate, pentaerythritol-modified triacrylate, sorbitol tetramethacrylate, sorbitol pentaacrylate, sorbitol hexaacrylate, oligoester acrylates and methacrylates, glycerol di- and tri-acrylate, 1,4-cyclohexane diacrylate, bisacrylates and bismethacrylates of polyethylene glycol, and mixtures thereof.

Under one embodiment, at least one of the one or more crosslinker is selected from the group consisting of trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, dipentaerythritol penta(meth)acrylate and ethoxylated iscyanuric acid tri(meth)acrylates.

Under one embodiment the crosslinkers comprise methacrylate groups. Example of such crosslinkers include dimethacrylates, trimethacrylates, tetramethacrylate, and higher methacrylates. Examples of such methacrylic crosslinkers include trimethylolpropane trimethacrylate, trimethylolethane trimethacrylate, tetramethylene glycol dimethacrylate, triethylene glycol dimethacrylate, pentaerythritol dimethacrylate, penta-erythritol trimethacrylate, dipentaerythritol dimethacrylate, dipentaerythritol tetramethacrylate, tripentaerythritol octamethacrylate, 1,3-butanediol dimethacrylate, sorbitol tetramethacrylate, oligoester methacrylates, bismethacrylates of polyethylene glycol having a molecular weight of from 200 to 1500, and mixtures thereof. For example, trimethylolpropane trimethacrylate is a composition consisting of, or comprising largely of, the compound of formula (CH₂=CMe-C(O)-O-CH₂)₃C-C₂H₅. It is a low volatility trifunctional monomer offering fast cure response in free radical polymerization.

Another suitable crosslinker is an alkoxylated crosslinker, with the formula (CH₂=CMe-C(O)-O-(AO)*ₓ*-CH₂-)₃C-R; wherein wherein R is a C₁ to C₆ alkyl group; AO is a small alkoxy group, such as an ethylene oxide, -CH₂-CH₂-O-, propylene oxide, -CH(CH₃)-CH₂-O-, -CH₂-CH₂-CH₂-O-, butylene oxide, and -CH(Et)-CH₂-O-; and wherein for each (CH₂=CMe-C(O)-O-(AO)*ₓ*-CH₂-) group *x* is independently 0, 1, 2, or 3. Using R = ethyl, and AO = ethylene oxide as an example, an exemplary alkoxylated crosslinker has a structure of formula 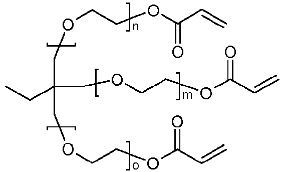 wherein *m, n,* and *o* are each independently 0, 1, 2, or 3.

Under one embodiment the composition of the present invention comprises (a) about 50 wt% to about 60 wt% of powder of one or more poly(C₁₋₁₂alkyl(meth)acrylate) polymers; (b) about 25 wt% to about 35 wt% of one or more (meth)acrylate oligomers, wherein the functionality of a (meth)acrylate oligomer is about 2 to about 30; (c) about 10 wt% to about 15 wt% of one or more (meth)acrylate monomers, and a crosslinker. This composition may optionally also include other ingredients.

Under one embodiment of the present invention, in this 50-60 wt% polymer : 25-35 wt% oligomer :10-15 wt% monomer composition, the polymers are insoluble in the composition; the oligomer comprises a urethane (meth)acrylate oligomer; and the monomers comprises a monomer that is hydroxyalkyl (meth)acrylate, cycloalkyl (meth)acrylate, and a mixture of both.

Under one embodiment the cross linker in this composition is a tri((meth)acrylate), and the composition further comprising a photoinitiator.

In addition to the above-described (meth)acrylate-based polymerizable materials, other polymerizable monomers, oligomers or polymers of monomers which contain at least one free radical polymerizable group in the molecule may be used without any limitations in the curable gel. Typical examples include esters of acrylic and methacrylic acid, herein termed (meth)acrylic ester. Specific but not limiting examples of mono (meth)acryloyl esters include methyl (meth)acrylate, ethyl (meth)acrylate, hydroxypropyl (meth)acrylate, butyl (meth)acrylates, hydroxy ethyl (meth)acrylates, butoxyethyl (meth)acrylate, diethylaminoethyl (meth)acrylate, 2-ethylhexyl (meth)acrylate, ethoxyethyl (meth)acrylate, t-butyl aminoethyl (meth)acrylate, methoxyethylene glycol (meth)acrylate, phosphoethyl (meth)acrylate, methoxy propyl (meth)acrylate, methoxy polyethylene glycol(meth)acrylate, phenoxyethylene glycol (meth)acrylate, phenoxypolyethylene glycol (meth)acrylate, 2-hydroxy-3-phenoxypropyl (meth)acrylate, 2-(meth)acryloxyethylsuccinic acid, 2-(meth)acryloylethylphthalic acid, 2-(meth)acryloyloxypropylphthalic acid, stearyl (meth)acrylate, isobornyl (meth)acrylate, 3-chloro-2-hydroxypropyl (meth)acrylates, tetrahydrofufuryl (meth)acrylate, methacryloyloxyethyl trimelilitc anhydride, (meth)acrylamides and allyl monomers. Specific but not limiting examples of difunctional (meth)acryloyl esters include 1,4 butane diol di(meth)acrylate, 1,6 hexananediol di(meth)acrylate, alkoxylated hexane diol di(meth)acrylate, 1,9 nonanediol di(meth)acrylate, 1,10 decanediol di(meth)acrylate, neopentyl glycol di(meth)acrylate, alkoxylated neopentyl glycol di(meth)acrylate, 2-methyl-1,8-octane diol di(meth)acrylate, cyclohaxane dimethanol di(meth)acrylate, glycerin di(meth)acrylate, ethylene glycol di(meth)acrylate, triethyleneglycol di(meth)acrylate, polyethylene glycol di(meth)acrylate, propylene glycol di(meth)acrylate, polypropylene glycol di(meth)acrylate, ethoxylated propylene glycol di(meth)acrylate, ethoxylated polypropylene glycol di(meth)acrylate, polyethoxypropoxy di(meth)acrylate, ethoxylated bisphenol A di(meth)acrylate, propoxylated bisphenol A di(meth)acrylate, propoxylated ethoxylated bisphenol A di(meth)acrylate, bisphenol A glycidyl methacrylate, tricyclodecanedimethanol di(meth)acrylate, glycerin di(meth)acrylate, ethoxylated glycerin di(meth)acrylate, bis acrylamides, bis allyl ethers and allyl (meth)acrylates. Examples of tri and or higher (meth)acryloyl esters include trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, dipentaerythritol penta(meth)acrylate and ethoxylated iscyanuric acid tri(meth)acrylates. Monomers containing acid groups may also be used including (meth)acrylic acid, bis(gyceryl dimethacrylate) pyromellitate, pyromellitic dimethacrylate, methacryloyloxyethyl phthalate, methacryloyloxyehtyl maleate, 2 hydroxyethyl methacrylate/succinate, 1,3 glyceryl dimethacylate maleate adduct, and 1,3 glyceryl dimethacrylate succinate adduct. Partially aminated monomers and oligomers may also be used. These are prepared by reaction of amines, preferably secondary amines, with some of the (meth)acryloyl groups of the multifunctional monomers or oligomers.

The photopolymerizable composition of the present invention for forming a cosmetic coating for nails also optionally comprises one or more photoinitiators. As described below, the photoinitiator is selected so that it is activated by photons of the wavelength associated with UV light of the UV lamp. Preferably, the photoinitiator should be active at the wavelength of UV light of UV lamps commonly found in nail salons.

Such photoinitiators may be selected from benzyl ketones, monomeric hydroxyl ketones, polymeric hydroxyl ketones, α-amino ketones, acyl phosphine oxides, metallocenes, benzophenone, and benzophenone derivatives. Specific examples of photoinitiators include 1-hydroxy-cyclohexylphenylketone; benzophenone; 2-benzyl-2-(dimethylamino)-1-(4-(4-morphorlinyl)phenyl)-1-butanone; 2,2-dimethoxy-2-phenyl acetophenone; 2-methyl-1-(4-methylthio)phenyl-2-(4-morphorlinyl)-1-propanone; 2,4,6-trimethylbenzoyldiphenylphosphine oxide; bis(2,4,6-trimethyl benzoyl)phenyl phosphine oxide; diphenyl-(2,4,6-trimethylbenzoyl) phosphine oxide; bis(2,6-dimethoxybenzoyl-2,4,4-trimethyl pentyl)phosphine oxide; 2-hydroxy-2-methyl-1-phenyl-propan-1-one; phenyl bis(2,4,6-trimethylbenzoyl) phosphine oxide; benzyl-dimethylketal; isopropylthioxanthone; bis(η⁵-2,4-cyclopentadien-1-yl)bis[2,6-difluoro-3-(1H-pyrrol-1-yl)phenyl]titanium), and mixtures of any of the foregoing.

Under one embodiment of the present invention the photopolymerizable composition comprises a single chemical compound that exhibits a photoinitiating properties. Under an alternative embodiment, the photoinitiator is a mixture of photoinitiators.

The photoinitiator is present in the photopolymerizable composition at amounts sufficient to be effective in aiding curing of the photopolymerizable composition. Such amounts may be determined empirically. The photopolymerizable composition comprises up to about 10 wt% of one or more photoinitiators. Under one embodiment, the photopolymerizable composition comprises about 0.5 to about 5.0 wt% of one or more photoinitiators.

The photopolymerizable composition of the present invention under one embodiment further comprises a small amount of a colorant or special effects pigment or a combination thereof.

One purpose of using pigment in the photopolymerizable composition is to provide a tint or a color to the formed cosmetic nail coating. The use of such color in the photopolymerizable composition may allow the technician to omit certain selected post treatment steps after the formation of the cosmetic nail coating.

Another purpose of using a pigment is to give a clear or colorless or whitish appearance of the cosmetic nail coating. The pigment may be used to address any yellowing of the cosmetic nail coating.

Yet another purpose of using a pigment is to provide a whitish appearance to the photopolymerizable composition, so that it appears as an attractive, clean product to the nail technician.

Examples of pigments may be incorporated into the photopolymerizable composition of the present invention include: annatto, caramel, carmine, β-carotene, potassium sodium copper chlorophyllin (chlorophyllin copper-complex), dihydroxyacetone, bismuth oxychloride, guaiazulene, iron oxides, ferric ammonium ferrocyanide, ferric ferrocyanide, chromium hydroxide green, chromium oxide greens, guanine, pyrophyllite, mica, silver, titanium dioxide, aluminum powder, bronze powder, copper powder, ultramarines, manganese violet, zinc oxide, luminescent zinc sulfide, FD&C Blue No. 1, D&C Blue No. 4, Iron Blue, D&C Brown No. 1, FD&C Green No. 3, D&C Green No. 5, D&C Green No. 6, D&C Green No. 8, D&C Orange No. 4, D&C Orange No. 5, D&C Orange No. 10, D&C Orange No. 11, FD&C Red No. 4, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, FD&C Red No. 40, D&C Violet No. 2, Ext. D&C Violet No. 2, FD&C Yellow No. 5, FD&C Yellow No. 6, D&C Yellow No. 7, Ext. D&C Yellow No. 7, D&C Yellow No. 8, D&C Yellow No. 10, D&C Yellow No. 11, and mixture of any of the foregoing. As will be recognized by the practitioner of the art, some of the pigments in the above list are better suited for use in the photopolymerizable composition than others, because they offer better composition stability of the photopolymerizable composition, and they do not interfere with the UV curing process.

Under one embodiment the photopolymerizable composition comprises the pigment is selected from the group consisting of ultramarine, manganese violet, zinc oxide, FD&C Blue No. 1, D&C Blue No. 4, Iron Blue, D&C Violet No. 2, and a mixture thereof.

Special effects pigment may be any pigment that gives either the photopolymerizable composition or the formed cured composition a special effect, such as an increased pearlescent, iridescent, shimmering, transparency or a complex effects. Examples of special effect pigments include titanated micas, mica based interference colors, mica coated with titanium dioxide and iron oxide, mica based gold pearls, mica based metallic pearls, mica based pearl pigments, bismuth oxychloride, synthetic mica based interference pearls, synthetic mica based white pigment, silicate based pearls, titanium oxide and tin oxide on silicate platelets, flaked aluminum powder, silver coated silicate flakes, and any combination of the foregoing.

The photopolymerizable composition of the present application may further comprise additional ingredients, including colorants, dyes, whiteners, perfumes, thixotropes, stabilizers, anti-oxidants, and like.

Any of above polymers, oligomers, monomers, crosslinkers, pigments, and other ingredients as provided by its manufacturer may contain small amounts of additives and impurities. The purity level of the ingredient may be above 90%. Alternatively, the purity level may be above 95%. Under some embodiments the purity level may be above 97%. Additives for monomers may include inhibitors such as hydroquinone, HQ, monomethyl ether quinone, MEHQ, isoascorbic acid, IA, butylated hydroxytoluene, BHT, and BHT adducts. Impurities may include isomers of the monomer, oligomers, unreacted starting materials, water, and solvent used in the formation of the monomers.

The photopolymerizable composition of the present invention for forming a cosmetic coating for nails is a viscous liquid. It has a similar consistency as a mixture of polymer powder and monomer liquid in a traditional two-part system that is middle of the open time.

Under one embodiment of the present invention the viscosity of the photopolymerizable composition is greater than about 400,000 millipascal-seconds as measured by a cone and plate rheometer. The viscosity is determined by a cone and plate rheometer using 40 mm 1° steel cone with the truncation gap of 31 microns operating at 2 to 10 s⁻¹ shear rate on a 500 mg sample. The temperature of the Peltier plate is set 25 °C, and the sample is conditioned to 25 °C. The cone and plate rheometer may be any suitable rheometer for measuring high viscosity fluids, such as TA Instruments AR1500EX Rheometer. The viscosity is determined from measurements at 5 points from 2 s⁻¹ to 10 s⁻¹ shear rates.

Under one embodiment the viscosity of the composition of the present invention is between 600,000 to 900,000 millipascal-seconds. Under another embodiment, the viscosity of the composition of the present invention is above 400,000 millipascal-seconds.

The present invention is also directed to the photopolymerizable composition wherein the composition exhibits a lower exotherm. The exotherm is the apparent rise in temperature due to the heat generated during the curing reaction caused by UV light. The exotherm is measured on a 0.5 gram sample formed on a glass substrate into a shape approximating a sculpted nail. The exotherm is measured by a thermocouple on the glass substrate to measure the approximate rise in temperatures that a client would feel during the curing process under the UV light.

The exotherm may be decreased by increasing the wt% of the polymer portion of the formulation. Further, the exotherm may be decreased by decreasing the wt% of the photoinitiators.

Under one embodiment of the present invention, the exotherm *(i.e.,* the peak temperature achieved during the reaction less the starting temperature) is less than about 30 °C (54 °F). Under another embodiment, the exotherm is less than about 20 °C (36 °F). Under still another embodiment the exotherm is less than about 15 °C (27 °C).

The photopolymerizable composition of the present invention under one embodiment is stable in a dark container at 49 °C for four months, or at 65 °C for 2 weeks. The stability may be tested in an enclosed container that does not let any UV light in. The term "stable" refers to the lack of change in the physical, chemical or esthetic properties of the photopolymerizable composition that would make the photopolymerizable composition unsuitable for sale to the consumer or would unsuitable for use by the technician. Exemplary changes of physical, chemical or esthetic properties of the photopolymerizable composition include polymerization, noticeable yellowing, noticeable rise in viscosity.

The photopolymerizable composition of the present invention may be prepared by any means used to add and blend high viscosity compositions. The components may be added together in any order that is convenient from engineering viewpoint. Addition of the components, blending of the components and filling appropriate containers may be done at elevated temperatures. Such temperatures should be less than temperatures which would initiate curing of the components.

The container in which the photopolymerizable composition of the present invention is sold may be any container capable of handling high viscosity compositions and of providing protection from UV light. One example of such a container is a wide-mouth jar. Another example of a suitable container is a tube container. Another example of a suitable container is a syringe container.

Another aspect of the present invention is a container comprising the previously described photopolymerizable composition, wherein the container is a tube container or a syringe container, wherein the container delivers a uniform bead of the composition via an orifice.

A tube container of the present application comprises the previously described photopolymerizable composition inside of a tube. The tube is a cylindrical, hollow piece with a round or oval profile, similar to tubes used to contain toothpaste, ointment, adhesives, caulk, and other viscous liquids. The tube has an orifice which is designed to deliver a bead of the photopolymerizable composition. Such an orifice may be a part of a nozzle, which may be optionally capped or closed.

A syringe container of the present application comprises the previously described photopolymerizable composition in a syringe. The syringe of the present invention comprises a plunger that fits tightly into a barrel. The plunger can be pushed along inside the barrel, allowing the syringe to expel the photopolymerizable composition through an orifice at the open end of the tube. The orifice is designed to deliver a bead of the photopolymerizable composition. Such an orifice may be a part of a nozzle, which may be optionally capped or closed.

The orifice is designed so that the technician is able to deliver a uniform bead to the nail of the client. The phrase "uniform bead" means that the weight of the bead formed does not vary by more than 10 wt% from another bead when forced through the orifice using the same conditions (the same pressure and same length of time) as subjectively applied by the technician.

Another aspect of the present invention is a method of use of the above-described photopolymerizable composition to form a cosmetic coating for a nail. There are three methods which may be used to form the cosmetic nail coating.

The first method of forming the cosmetic nail coating comprises the steps of placing the above-described composition onto a nail of the client; and exposing the composition to UV light.

The placement of the bead of the photopolymerizable composition may be performed by the technician directly by squeezing it from a tube container or pushing it from a syringe with a plunger. Alternatively, the bead of the photopolymerizable composition may be done with the help of an acrylic or gel brush, such as a No. 8, 10, or 12 brush.

After the placement of the bead of the photopolymerizable composition onto the nail, the technician works the composition to move it into a desired location and form it into a desired shape.

After the composition is shaped, the composition, along with finger and the hand of the client, is exposed to UV light to cure the composition. A suitable UV light may be natural sunlight. Another suitable UV light may be a UV lamp, such as a 36 watt lamp commonly used in many nail salons. Such a UV lamp may operate at any wavelength required to cure the photopolymerizable composition, such as between 320 nm and 420 nm. The exposure time should be as long enough to allow for curing of the photopolymerizable composition. This may be 5 seconds to 6 minutes.

The term "UV lamp" is meant to be interpreted broadly. It refers to any source of electromagnetic radiation that exhibits light in the 320 nm to 420 nm range at sufficient enough strength to cure the composition of the present invention. The term "UV lamp" includes traditional UV lamps that contain fluorescent lamps, such as compact fluorescent light bulbs, that give off UV light in the above-described ranges. The term "UV lamp" also refers to newer sources of light or UV radiation, such as light-emitting diode lamps (commonly referred to as "LED lamps") that emit electromagnetic radiation which includes UV light in the 320 nm to 420 nm range at sufficient enough strength to cure the composition of the present invention. The term "UV lamp" also refers to any other type of source of light that comprises UV light in the 320 nm to 420 nm range at sufficient enough strength to cure the composition of the present invention.

The second method of forming an acrylic nail comprises the steps of contacting the photopolymerizable composition with a liquid to create a blend; placing the blend onto a nail; and exposing the blend to UV light.

The second method is similar to the first method, except that the photopolymerizable composition is not placed on the nail neat, but is first wetted with a liquid. According to the second method, the technician uses the liquid to help in lowering the viscosity of the composition.

The liquid of this method is selected from the group consisting of an acrylic nail monomer, solvent, oil, slip agent, and mixtures thereof. The solvent must be miscible with the photopolymerizable composition. The phrase "acrylic nail monomer" or the phrase "monomer liquid" means a liquid that is used by technicians to prepare acrylic nails.

The term "liquid" when referring to the monomer liquid means a liquid composition that is sufficiently homogeneous to be used for preparation of acrylic nails. The liquid may be a uniform clear solution, a liquid that displays a Tyndall effect, a colloid, or a suspension in which any fine particles of the suspension do not precipitate during storage time. The liquid may be colorless, or it may be colored.

The third method of forming an acrylic nail comprises the steps of placing the photopolymerizable composition onto a nail; contacting the composition with a liquid; and exposing the mixture to UV light.

The third method is similar to the first method, except that the photopolymerizable composition is placed on the nail neat, and is then wetted with the liquid. According to the third method, the technician uses the liquid to help in lowering the viscosity of the composition after the composition is placed on the nail. The liquid of the third method is as described above for the second method.

The terms "cure", "curing", and like, are used herein are similar to those in the nail art, and are broadly encompassing terms. These term refer to any portion of, or the entire process of polymerization which is experienced under the UV light.

### Examples

A typical exemplary photopolymerizable composition for forming a cosmetic coating for nails has the following ingredients:

| Chemical name | % |
|---|---|
| (Meth)acrylic polymer | 50-60 |
| Urethane (meth)acrylate oligomer | 25-35 |
| Mixture of hydroxyalkyl (meth)acrylate and cycloalkyl (meth)acrylate | 10-15 |
| Tri((meth)acrylate) | 1 |
| Photoinitiator | 2 |
| Antioxidant | <1 |
| Colorant | <0.05 |

For each of the formulations, the above ingredients were added together and mixed until the composition was homogeneous. The order of addition of the ingredients did not show any appreciable differences in the performance of the composition.

A sample bead of the above formulation was placed on a microscope slide. The bead had the viscosity and workability of a typical traditional two part system about 4 minutes after the monomer liquid and the polymer have been mixed. The open time appeared to be much longer than typically necessary by even the most inexperienced nail technician. The bead was spread and exposed to a UV lamp, to yield a hard, durable substance.

### Viscosity measurements

The viscosities of samples of the above formulation (prior to curing) were measured using a TA Instruments AR1500EX Rheometer using a cone and plate setup, with a 40mm 1° steel cone. The temperature of the Peltier plate was set 25 °C and the instrument was calibrated prior the use. The sample (0.5 ± 0.05 g) was loaded in the center of the plate, the head was lowered to the truncation gap of 31 microns, and the sample was conditioned to 25 °C. The measurements were taken under steady state flow conditions, where the temperature and truncation gap were held constant throughout the run. The viscosity of the samples was measured at 5 points during the analysis, with each data point collected at a different, increasing shear rate. The initial shear rate is 2 s⁻¹ and each subsequent measurement was taken as the shear rate was increased by increments of 2 s⁻¹ until the final shear rate of 10 s⁻¹ was reached. The viscosity obtained at the 10 s⁻¹ shear rate was recorded as the final viscosity of the sample. The viscosity of a typical sample was between 600,000 to 900,000 millipascal-seconds.

### Exotherm Measurements

A 0.5 g sample of the above formulation was compared to a 0.5 g sample of a mixture of the same gel and a polymer powder, and the amount of photoinitiators was adjusted so that both samples had the same quantity of photoiniators. The samples were applied to a themocouple on a glass slide. After photoinitiation, there was a 19 °C rise in the sample without the polymer powder but only a 12 °C rise in the sample with the polymer powder.

### Stability Testing

An accelerated thermal stability test was completed on samples of two formulations of a one-part acrylic nail formula of the present invention. Formulation A is similar to that of Formulation B, except for containing a different polymer powder. A sample of each formulation was held at room temperature, 49 °C, and 65 °C. The samples were monitored at various time intervals and compared to one another to observe if any changes in physical state or performance of the samples could be noted; namely yellowing and polymerization. Under this test, four months at 49 °C and two weeks at 65 °C are the approximate equivalent to having a sample age two years at room temperature; any sample that remained relatively unchanged after 2 weeks at 65 °C was said to pass stability testing.

The samples were contained in ½ oz (15 mL) polypropylene containers fitted with a foil seal lid with a 15% head space and were monitored at 0 days, 1 day, 3 days, 1 week, and 2 weeks for any observable differences at room temperature, 49 °C, and 65 °C. Within 3 days, Formula A had yellowed at both 49 °C and 65 °C. The 65 °C sample was much more yellow than the 49 °C sample and the 49 °C sample was yellow but not as severely as the 65 °C sample. The room temperature sample did not change color. The 65 °C sample had also polymerized during this time interval and the 49 °C sample had thickened. The room temperature sample remained unchanged in thickness during the same time interval. The 49 °C sample had polymerized by the second week. Formula B remained unchanged over the same temperatures and times.

While the present invention has been described with reference to several embodiments, which embodiments have been set forth in considerable detail for the purposes of making a complete disclosure of the invention, such embodiments are merely exemplary and are not intended to be limiting or represent an exhaustive enumeration of all aspects of the invention. The scope of the invention is to be determined from the claims appended hereto.

## Claims

1. A photopolymerizable composition for forming a cosmetic coating for nails comprising:
(a) about 50 wt% to about 60 wt% of powder of one or more poly(C₁₋₁₂alkyl(meth)acrylate) polymers;
(b) about 25 wt% to about 35 wt% of one or more (meth)acrylate oligomers, wherein the functionality of a (meth)acrylate oligomer is about 2 to about 30;
(c) about 10 wt% to about 15 wt% of one or more (meth)acrylate monomers; and
(d) one or more crosslinkers;
wherein all wt% are with respect to the photopolymerizable composition.

2. The composition of claim 1, wherein the one or more poly(C₁₋₁₂alkyl(meth)acrylate) polymers are insoluble in the composition; the one or more (meth)acrylate oligomers comprise a urethane (meth)acrylate oligomer; and the one or more (meth)acrylate monomers comprise a monomer selected from the group consisting of hydroxyalkyl (meth)acrylate, cycloalkyl (meth)acrylate, and a mixture thereof;
optionally wherein the one or more crosslinkers comprises a tri((meth)acrylate), and further comprising a photoinitiator.

3. The composition of claim 1, wherein at least one of the one or more polymers is selected from the group consisting of poly(methyl methacrylate), poly(ethyl methacrylate), and a mixture thereof.

4. The composition of claim 1, wherein at least one of the one or more polymers comprises poly(methyl methacrylate) particles, wherein the mean particle size of poly(methyl methacrylate) particles is less than about 100 micrometers; optionally wherein the mean particle size of poly(methyl methacrylate) particles is less than about 50 micrometers; optionally wherein the mean particle size of poly(methyl methacrylate) particles is less than about 10 micrometers; optionally wherein the mean particle size of poly(methyl methacrylate) particles is less than about 5 micrometers.

5. The composition of claim 1, wherein the (meth)acrylate oligomer is selected from the group consisting of urethane (meth)acrylate, epoxy (meth)acrylate, epoxy urethane (meth)acrylate, (meth)acrylated acrylate, (meth)acrylated polyether, (meth)acrylated polycarbonate, (meth)acrylated cellulose, (meth)acrylated butadiene, (meth)acrylated styrene, polyester (meth)acrylate, polyester urethane (meth)acrylate, polyether urethane (meth)acrylate, polybutadiene urethane (meth)acrylate, and a mixture thereof.

6. The composition of claim 1, wherein at least one of the one or more (meth)acrylate monomers comprises a hydroxyl-containing (meth)acrylate monomer.

7. The composition of claim 1, wherein at least one of the one or more (meth)acrylate monomers comprise a methacrylate selected from the group consisting of hydroxypropyl methacrylate, hydroxyethyl methacrylate, ethyl methacrylate, propyl methacrylate, butyl methacrylate, isobutyl methacrylate, sec-butyl methacrylate, tetrahydrofurfuryl methacrylate, caprolactone methacrylate, methacroyloxyethyl maleate, 2-hydroxyethyl methacrylate/succinate, phthalic acid monoethyl methacrylate, or a mixture thereof.

8. The composition of claim 1, wherein at least one of the one or more crosslinkers is selected from the group consisting of tri(meth)acrylate, tetra(meth)acrylate, penta(meth)acrylate, and a mixture thereof; or
wherein at least one of the one or more crosslinker is an alkoxylated crosslinker of formula (CH₂=CMe-C(O)-O-(AO)ₓ-CH₂-)₃C-R;
wherein R is a C₁ to C₆ alkyl group;
AO is an alkoxy group selected from the group consisting of ethylene oxide, -CH₂-CH₂-O-, propylene oxide, -CH(CH₃)-CH₂-O-, -CH₂-CH₂-CH₂-O-, butylene oxide, and -CH(Et)-CH₂-O-; and
wherein for each CH₂=CMe-C(O)-O-(AO)ₓ-CH₂- group *x* is independently 0, 1, 2, or 3; or
wherein one crosslinker is ethoxylated crosslinker of formula (CH₂=CMe-C(O)-O-(CH₂-CH₂-O)*ₓ*-CH₂-)₃C-C₂H₅;
wherein for each CH₂=CMe-C(O)-O-(CH₂-CH₂-O)*ₓ*-CH₂- group *x* is independently 0, 1, 2, or 3; or
wherein at least one of the one or more crosslinker is selected from the group consisting of trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, dipentaerythritol penta(meth)acrylate and ethoxylated iscyanuric acid tri(meth)acrylates.

9. The composition of claim 1, further comprising one or more photoinitiators, optionally wherein at least one of the one or more photoinitiators is selected from the group consisting of 1-hydroxy-cyclohexylphenylketone; benzophenone; 2-benzyl-2-(dimethylamino)-1-(4-(4-morphorlinyl)phenyl)-1-butanone; 2,2-dimethoxy-2-phenyl acetophenone; 2-methyl-1-(4-methylthio)phenyl-2-(4-morphorlinyl)-1-propanone; 2,4,6-trimethylbenzoyldiphenylphosphine oxide; bis(2,4,6-trimethyl benzoyl)phenyl phosphine oxide; diphenyl-(2,4,6-trimethylbenzoyl) phosphine oxide; bis(2,6-dimethoxybenzoyl-2,4,4-trimethyl pentyl)phosphine oxide; 2-hydroxy-2-methyl-1-phenyl-1-propanone; phenyl bis(2,4,6-trimethylbenzoyl) phosphine oxide; benzyl-dimethylketal; isopropylthioxanthone; bis(η⁵-2,4-cyclopentadien-1-yl)bis[2,6-difluoro-3-(1H-pyrrol-1-yl)phenyl]titanium); α,α-dimethoxy α-phenyl acetophenone; ethyl (2,4,6-trimethyl benzoyl) phenyl phosphinate; phenyl (2,4,6-trimethyl benzoyl) phenyl phosphinate, methyl benzoyl formate, and mixtures thereof.

10. The composition of claim 1, wherein the composition further comprises a colorant or special effects pigment or a combination thereof, optionally wherein the colorant is selected from the group consisting of ultramarine, manganese violet, zinc oxide, FD&C Blue No. 1, D&C Blue No. 4, Iron Blue, D&C Violet No. 2, and a mixture thereof.

11. The composition of claim 1, wherein the viscosity of the composition is greater than 400,000 millipascal-seconds (400,000 centipoise) at 25 °C as determined by a cone and plate rheometer using 40 mm 1° steel cone with the truncation gap of 31 microns operating at a 10 s⁻¹ shear rate on a 500 mg sample.

12. A method of forming a cosmetic nail coating comprising the steps of:
(a) placing the composition of claim 9 onto a nail; and
(b) exposing the composition to UV light; or
(a) contacting the composition of claim 1 with a liquid selected from the group consisting of an acrylic nail monomer, solvent, oil, slip agent, photoinitiator and mixtures thereof to create a blend;
(b) placing the blend onto a nail; and
(c) exposing the blend to UV light; or
(a) placing the composition of claim 1 onto a nail;
(b) contacting the composition with a liquid selected from the group consisting of an acrylic nail monomer, solvent, oil, photoinitiator and mixtures thereof; and
(c) exposing the mixture to UV light.

13. A container containing the composition of claim 1, wherein the container is a tube container or a syringe container, and wherein the container delivers a uniform bead of the composition.

## Patentansprüche

1. Photopolymerisierbare Zusammensetzung zum Bilden einer kosmetischen Beschichtung für Nägel, umfassend:
(a) etwa 50 Gew.-% bis etwa 60 Gew.-% Pulver aus einem oder mehreren Poly(C₁₋₁₂-alkyl(meth)acrylat)-Polymeren;
(b) etwa 25 Gew.-% bis etwa 35 Gew.-% eines oder mehrerer (Meth)acrylat-Oligomere, wobei die Funktionalität eines (Meth)acrylat-Oligomers etwa 2 bis etwa 30 beträgt;
(c) etwa 10 Gew.-% bis etwa 15 Gew.-% eines oder mehrerer (Meth)acrylat-Monomere; und
(d) ein oder mehrere Vernetzer;
wobei alle Gew.-% auf die photopolymerisierbare Zusammensetzung bezogen sind.

2. Zusammensetzung nach Anspruch 1, wobei das eine oder die mehreren Poly(C₁₋₁₂-alkyl(meth)acrylat)-Polymere in der Zusammensetzung unlöslich sind; das eine oder die mehreren (Meth)acrylat-Oligomere ein Urethan-(Meth)acrylat-Oligomer umfassen; und das eine oder die mehreren (Meth)acrylat-Monomere ein Monomer umfassen, ausgewählt aus der Gruppe bestehend aus Hydroxyalkyl(meth)acrylat, Cycloalkyl(meth)acrylat und einer Mischung davon;
optional, wobei der eine oder die mehreren Vernetzer ein Tri((meth)acrylat) umfassen und ferner einen Photoinitiator umfassen.

3. Zusammensetzung nach Anspruch 1, wobei mindestens eines der einen oder mehreren Polymere ausgewählt ist aus der Gruppe bestehend aus Poly(methylmethacrylat), Poly(ethylmethacrylat) und einer Mischung davon.

4. Zusammensetzung nach Anspruch 1, wobei mindestens eines der einen oder mehreren Polymere Poly(methylmethacrylat)-Partikel umfasst, wobei die durchschnittliche Partikelgröße von Poly(methylmethacrylat)-Partikeln weniger als etwa 100 Mikrometer beträgt; optional, wobei die durchschnittliche Partikelgröße von Poly(methylmethacrylat)-Partikeln weniger als etwa 50 Mikrometer beträgt; optional, wobei die durchschnittliche Partikelgröße von Poly(methylmethacrylat)-Partikeln weniger als etwa 10 Mikrometer beträgt; optional, wobei die durchschnittliche Partikelgröße von Poly(methylmethacrylat)-Partikeln weniger als etwa 5 Mikrometer beträgt.

5. Zusammensetzung nach Anspruch 1, wobei das (Meth)acrylat-Oligomer ausgewählt ist aus der Gruppe bestehend aus Urethan(meth)acrylat, Epoxy(meth)acrylat, Epoxyurethan(meth)acrylat, (meth)acryliertem Acrylat, (meth)acryliertem Polyether, (meth)acryliertem Polycarbonat, (meth)acrylierter Cellulose, (meth)acryliertem Butadien, (meth)acryliertem Styrol, Polyester(meth)acrylat, Polyesterurethan(meth)acrylat, Polyetherurethan(meth)acrylat, Polybutadienurethan(meth)acrylat und einer Mischung davon.

6. Zusammensetzung nach Anspruch 1, wobei mindestens eines der einen oder mehreren (Meth)acrylat-Monomere ein hydroxylhaltiges (Meth)acrylat-Monomer umfasst.

7. Zusammensetzung nach Anspruch 1, wobei mindestens eines der einen oder mehreren (Meth)acrylat-Monomere ein Methacrylat umfasst, ausgewählt aus der Gruppe bestehend aus Hydroxypropylmethacrylat, Hydroxyethylmethacrylat, Ethylmethacrylat, Propylmethacrylat, Butylmethacrylat, Isobutylmethacrylat, sec-Butylmethacrylat, Tetrahydrofurfurylmethacrylat, Caprolactonmethacrylat, Methacroyloxyethylmaleat, 2-Hydroxyethylmethacrylat/succinat, Phthalsäuremonoethylmethacrylat oder einer Mischung davon.

8. Zusammensetzung nach Anspruch 1, wobei mindestens einer der einen oder mehreren Vernetzer ausgewählt ist aus der Gruppe bestehend aus Tri(meth)acrylat, Tetra(meth)acrylat, Penta(meth)acrylat und einer Mischung davon; oder
wobei mindestens einer der einen oder mehreren Vernetzer ein alkoxylierter Vernetzer der Formel (CH₂=CMe-C(O)-O-(AO)*ₓ*-CH₂-)₃C-R ist;
wobei R eine C₁ bis C₆-Alkylgruppe ist;
AO eine Alkoxygruppe ist, ausgewählt aus der Gruppe bestehend aus Ethylenoxid, -CH₂-CH₂-O-, Propylenoxid, -CH(CH₃)-CH₂-O-, -CH₂-CH₂-CH₂-O-, Butylenoxid und -CH(Et)-CH₂-O-; und
wobei für jede CH₂=CMe-C(O)-O-(AO)*ₓ*-CH₂-Gruppe x unabhängig 0, 1, 2 oder 3 ist; oder
wobei ein Vernetzer ein ethoxylierter Vernetzer der Formel (CH₂=CMe-C(O)-O-(CH₂-CH₂-O)*ₓ*-CH₂-)₃C-C₂H₅ ist;
wobei für jede CH₂=CMe-C(O)-O-(CH₂-CH₂-O)*ₓ*CH₂-Gruppe x unabhängig 0, 1, 2 oder 3 ist; oder
wobei mindestens einer der einen oder mehreren Vernetzer ausgewählt ist aus der Gruppe bestehend aus Trimethylolpropantri(meth)acrylat, ethoxyliertem Glycerintri(meth)acrylat, ethoxyliertem Trimethylolpropantri(meth)acrylat, Ditrimethylolpropantetra(meth)acrylat, Pentaerythritoltri(meth)acrylat, Pentaerythritoltetra(meth)acrylat, propoxyliertem Pentaerythritoltetra(meth)acrylat, ethoxyliertes Pentaerythritoltetra(meth)acrylat, Dipentaerythritolhexa(meth)acrylat, Dipentaerythritolpenta(meth)acrylat und ethoxylierte Iscyanursäuretri(meth)acrylate.

9. Zusammensetzung nach Anspruch 1, ferner umfassend einen oder mehrere Photoinitiatoren, optional, wobei mindestens einer der einen oder mehreren Photoinitiatoren ausgewählt ist aus der Gruppe bestehend aus 1-Hydroxycyclohexylphenylketon, Benzophenon, 2-Benzyl-2-(dimethylamino)-1-(4-(4-morpholinyl)phenyl)-1-butanon; 2,2-Dimethoxy-2-phenylacetophenon; 2-Methyl-1-(4-methylthio)phenyl-2-(4-morpholinyl)-1-propanon; 2,4,6-Trimethylbenzoyldiphenylphosphinoxid; Bis(2,4,6-trimethylbenzoyl)phenylphosphinoxid; Diphenyl-(2,4,6-trimethylbenzoyl)phosphinoxid; Bis(2,6-dimethoxybenzoyl-2,4,4-trimethylpentyl)phosphinoxid; 2-Hydroxy-2-methyl-1-phenyl-1-propanon; Phenylbis(2,4,6-trimethylbenzoyl)phosphinoxid; Benzyldimethylketal; Isopropylthioxanthon; Bis(⁵η-2,4-cyclopentadien-1-yl)bis[2,6-difluor-3-(1H-pyrrol-1-yl)phenyl]titan); α,α-Dimethoxy-α-phenylacetophenon; Ethyl(2,4,6-trimethylbenzoyl)phenylphosphinat; Phenyl(2,4,6-trimethylbenzoyl)phenylphosphinat, Methylbenzoylformiat und Mischungen davon.

10. Zusammensetzung nach Anspruch 1, wobei die Zusammensetzung ferner ein Färbemittel oder ein Spezialeffektpigment oder eine Kombination davon umfasst, optional, wobei das Färbemittel ausgewählt ist aus der Gruppe bestehend aus Ultramarin, Manganviolett, Zinkoxid, FD&C Blue Nr. 1, D&C Blue Nr. 4, Eisenblau, D&C Violet Nr. 2 und einer Mischung davon.

11. Zusammensetzung nach Anspruch 1, wobei die Viskosität der Zusammensetzung größer als 400.000 Millipascalsekunden (400.000 Centipoise) bei 25 °C beträgt, wie mittels eines Kegel-Platten-Rheometers unter Verwendung eines 40-mm-1°-Stahlkegels mit dem Stumpfspalt von 31 Mikrometern bei einer Schergeschwindigkeit von 10 s⁻¹ an einer 500-mg-Probe bestimmt.

12. Verfahren zum Bilden einer kosmetischen Nagelbeschichtung, umfassend die Schritte aus:
(a) Platzieren der Zusammensetzung nach Anspruch 9 auf einen Nagel; und
(b) Aussetzen der Zusammensetzung gegenüber UV-Licht; oder
(a) Inkontaktbringen der Zusammensetzung nach Anspruch 1 mit einer Flüssigkeit, ausgewählt aus der Gruppe bestehend aus einem Acrylnagel-Monomer, Lösungsmittel, Öl, Gleitmittel, Photoinitiator und Mischungen davon, um ein Gemisch zu erzeugen;
(b) Platzieren des Gemischs auf einen Nagel; und
(c) Aussetzen des Gemischs gegenüber UV-Licht; oder
(a) Platzieren der Zusammensetzung nach Anspruch 1 auf einen Nagel;
(b) Inkontaktbringen der Zusammensetzung mit einer Flüssigkeit, ausgewählt aus der Gruppe bestehend aus einem Acrylnagel-Monomer, Lösungsmittel, Öl, Photoinitiator und Mischungen davon; und
(c) Aussetzen der Mischung gegenüber UV-Licht.

13. Behälter, enthaltend die Zusammensetzung nach Anspruch 1, wobei der Behälter ein Tubenbehälter oder ein Spritzenbehälter ist und wobei der Behälter einen gleichmäßigen Tropfen der Zusammensetzung freisetzt.

## Revendications

1. Composition photopolymérisable pour former un revêtement cosmétique pour ongles comportant :
(a) environ 50 % en poids à environ 60 % en poids de poudre d'un ou plusieurs polymères de poly (C₁-₁₂alkyl(méth)acrylate) ;
(b) environ 25 % en poids à environ 35 % en poids d'un ou plusieurs oligomères de (méth)acrylate, dans laquelle la fonctionnalité d'un oligomère de (méth)acrylate est d'environ 2 à environ 30 ;
(c) environ 10 % en poids à environ 15 % en poids d'un ou plusieurs monomères de (méth)acrylate ; et
(d) un ou plusieurs réticulants ;
dans laquelle tous les % en poids sont par rapport à la composition photopolymérisable.

2. Composition selon la revendication 1, dans laquelle les un ou plusieurs polymères de poly(C₁-₁₂alkyl(méth)acrylate) sont insolubles dans la composition ; les un ou plusieurs oligomères de (méth)acrylate comportent un oligomère de (méth)acrylate d'uréthane ; et les un ou plusieurs monomères de (méth)acrylate comportent un monomère choisi dans le groupe constitué par le (méth)acrylate d'hydroxyalkyle, le (méth)acrylate de cycloalkyle, et un mélange de ceux-ci ;
facultativement dans laquelle les un ou plusieurs réticulants comportent un tri((méth)acrylate), et comportant en outre un photoinitiateur.

3. Composition selon la revendication 1, dans laquelle au moins l'un des un ou plusieurs polymères est choisi dans le groupe constitué par le poly(méthacrylate de méthyle), le poly(méthacrylate d'éthyle), et un mélange de ceux-ci.

4. Composition selon la revendication 1, dans laquelle au moins l'un des un ou plusieurs polymères comporte des particules de poly(méthacrylate de méthyle), dans laquelle la taille moyenne de particule des particules de poly(méthacrylate de méthyle) est inférieure à environ 100 micromètres ; facultativement dans laquelle la taille moyenne de particule des particules de poly(méthacrylate de méthyle) est inférieure à environ 50 micromètres ; facultativement dans laquelle la taille moyenne de particule des particules de poly(méthacrylate de méthyle) est inférieure à environ 10 micromètres ; facultativement dans laquelle la taille moyenne de particule des particules de poly(méthacrylate de méthyle) est inférieure à environ 5 micromètres.

5. Composition selon la revendication 1, dans laquelle l'oligomère de (méth)acrylate est choisi dans le groupe constitué par les (méth)acrylate d'uréthane, (méth)acrylate d'époxy, (méth)acrylate d'époxy uréthane, acrylate (méth)acrylé, polyéther (méth)acrylé, polycarbonate (méth)acrylé, cellulose (méth)acrylée, butadiène (méth)acrylé, styrène (méth)acrylé, (méth)acrylate de polyester, (méth)acrylate de polyester uréthane, (méth)acrylate de polyether uréthane, (méth)acrylate de polybutadiène uréthane, et un mélange de ceux-ci.

6. Composition selon la revendication 1, dans laquelle au moins l'un des un ou plusieurs monomères de (méth)acrylate comporte un monomère de (méth)acrylate contenant de l'hydroxyle.

7. Composition selon la revendication 1, dans laquelle au moins l'un des un ou plusieurs monomères de (méth)acrylate comporte un méthacrylate choisi dans le groupe constitué par les méthacrylate d'hydroxypropyle, méthacrylate d'hydroxyéthyle, méthacrylate d'éthyle, méthacrylate de propyle, méthacrylate de butyle, méthacrylate d'isobutyle, méthacrylate de sec-butyle, méthacrylate de tétrahydrofurfuryle, méthacrylate de caprolactone, maléate de méthacroyloxyéthyle, méthacrylate/succinate de 2-hydroxyéthyle, méthacrylate de monoéthyl d'acide phtalique, ou un mélange de ceux-ci.

8. Composition selon la revendication 1, dans laquelle au moins l'un des un ou plusieurs réticulants est choisi dans le groupe constitué par les tri(méth)acrylate, tétra(méth)acrylate, penta(méth)acrylate, et un mélange de ceux-ci ; ou
dans laquelle au moins l'un des un ou plusieurs réticulants est un réticulant alcoxylé de formule (CH₂=CMe-C(O)-O-(AO)ₓ-CH₂-)₃C-R ;
dans laquelle R est un groupe alkyle en C₁ à C₆ ;
AO est un groupe alcoxy choisi dans le groupe constitué par les oxyde d'éthylène, -CH₂-CH₂-O-, oxyde de propylène, - CH(CH₃)-CH₂-O-, -CH₂-CH₂-CH₂-O-, oxyde de butylène, et - CH(Et)-CH₂-O- ; et
dans laquelle pour chaque groupe CH₂=CMe-C(O)-O-(AO)ₓ-CH₂- x est indépendamment 0, 1, 2 ou 3 ; ou
dans laquelle un réticulant est un réticulant éthoxylé de formule (CH₂=CMe-C(O)-O-(CH₂-CH₂-O)ₓ-CH₂-)₃C-C₂H₅ ;
dans laquelle pour chaque groupe CH₂=CMe-C(O)-O-(CH₂-CH₂-O)ₓ-CH₂- x est indépendamment 0, 1, 2 ou 3 ; ou
dans laquelle au moins l'un des un ou plusieurs réticulants est choisi dans le groupe constitué par les tri(méth)acrylate de triméthylol propane, tri(méth)acrylate de glycérine éthoxylé, tri(méth)acrylate de triméthylolpropane éthoxylé, tétra(méth)acrylate de ditriméthylol propane, tri(méth)acrylate de pentaérythritol, tétra(méth)acrylate de pentaérythritol, tétra(méth)acrylate de pentaérythritol propoxylé, tétra(méth)acrylate de pentaérythritol éthoxylé, hexa(méth)acrylate de dipentaérythritol, penta(méth)acrylate de dipentaérythritol et tri(méth)acrylates d'acide isocyanurique éthoxylé.

9. Composition selon la revendication 1, comportant en outre un ou plusieurs photoinitiateurs, facultativement dans laquelle au moins l'un des un ou plusieurs photoinitiateurs est choisi dans le groupe constitué par 1-hydroxy-cyclohexylphénylcétone ; benzophénone ; 2-benzyl-2-(diméthylamino)-1-(4-(4-morphorlinyl)phényl)-1-butanone ; 2,2-diméthoxy-2-phényl acétophénone ; 2-méthyl-1-(4-méthylthio)phényl-2-(4-morphorlinyl)-1-propanone ; oxyde de 2,4,6-triméthylbenzoyldiphényl-phosphine ; oxyde de bis(2,4,6-triméthylbenzoyl)phénylphosphine ; oxyde de diphényl-(2,4,6-triméthylbenzoyl)phosphine ; oxyde de bis(2,6-diméthoxybenzoyl-2,4,4-triméthylpentyle)phosphine ; 2-hydroxy-2-méthyl-1-phényl-1-propanone ; oxyde de phényl bis(2,4,6-triméthylbenzoyl)phosphine ; benzyl-diméthylcétal ; isopropylthioxanthone ; bis(η⁵-2,4-cyclopentadiène-1-yl)bis[2,6-difluoro-3-(1H-pyrrol-1-yl)phényl]titane) ; α,α-diméthoxy α-phényl acétophénone ; éthyl (2,4,6-triméthyl benzoyl) phényl phosphinate ; phényl (2,4,6-triméthyl benzoyl) phényl phosphinate, méthyl benzoyl formate, et des mélanges de ceux-ci.

10. Composition selon la revendication 1, dans laquelle la composition comporte en outre un colorant ou pigment à effets spéciaux ou une combinaison de ceux-ci, facultativement dans laquelle le colorant est choisi dans le groupe constitué par l'ultramarin, le violet de manganèse, l'oxyde de zinc, le bleu FD&C n°1, le bleu D&C n°4, le bleu fer, le violet D&C n°2, et un mélange de ceux-ci.

11. Composition selon la revendication 1, dans laquelle la viscosité de la composition est supérieure à 400 000 millipascal-secondes (400 000 centipoises) à 25 °C telle que déterminée par un rhéomètre cône-plan utilisant un cône en acier de 40 mm 1° avec l'écart de troncation de 31 microns fonctionnant à une vitesse de cisaillement de 10 s⁻¹ sur un échantillon de 500 mg.

12. Procédé de formation d'un revêtement cosmétique pour ongles comportant les étapes de :
(a) placement de la composition selon la revendication 9 sur un ongle ; et
(b) exposition de la composition à la lumière UV ; ou
(a) mise en contact de la composition selon la revendication 1 avec un liquide choisi dans le groupe constitué par un monomère pour ongle acrylique, un solvant, une huile, un agent glissant, un photoinitiateur et des mélanges de ceux-ci pour créer un mélange homogène ;
(b) placement du mélange homogène sur un ongle ; et
(c) exposition du mélange homogène à la lumière UV ; ou
(a) placement de la composition selon la revendication 1 sur un ongle ;
(b) mise en contact de la composition avec un liquide choisi dans le groupe constitué par un monomère pour ongle acrylique, un solvant, une huile, un photoinitiateur et des mélanges de ceux-ci ; et
(c) exposition du mélange à la lumière UV.

13. Récipient contenant la composition selon la revendication 1, dans lequel le récipient est un récipient tubulaire ou un récipient de type seringue, et dans lequel le récipient délivre une boule uniforme de la composition.
